# EUROPEAN PATENT APPLICATION

(11) **EP 2 412 819 A1**
(43) Date of publication of application: **01.02.2012**
(21) Application number: 10171276.8
(22) Date of filing: 29.07.2010
(51) Int. Cl.: C12Q 1/18

(54) **Methods and uses relating to the identification of compound associated with bacterial infection**

(71) Applicant: Sanofi-Aventis Deutschland GmbH, 65929 Frankfurt am Main (DE)
(72) Inventor: The designation of the inventor has not yet been filed

(57) **Abstract**

The present invention relates to a method of identifying a compound useful for the treating, reducing or preventing a pathogenic infection caused by a microorganism and to the use of the PqsD protein or a functional fragment or variant thereof for the identification of a compound having said effect.

## Description

The present invention relates to a method of identifying a compound associated with pathogenic infection and the use of PqsD protein for identifying a compound associated with pathogenic infection.

Cell-cell communication or intercellular signalling in bacteria, also designated "quorum sensing (QS)", enables coordination of gene expression in a bacterial population. This communication is dependent on formation of diffusible signal molecules which are released into the extracellular region where they, as soon as they exceed a specific threshold, effect a coordinated change of expression of QS-dependent target genes. Known effects in Gram-positive as well as Gram-negative bacteria which are regulated by quorum sensing are an increase of mobility of plasmids, formation of biofilm and enhanced virulence (Deziel E. et al., Proc. Natl. Acad. Sci. USA 2004, 101: 1339-1344). The development of inhibitors of QS as a potential approach for new anti-infective agents is momentarily heavily investigated (Ni N. et al., Med. Res. Rev. 2009; 29: 65-124).

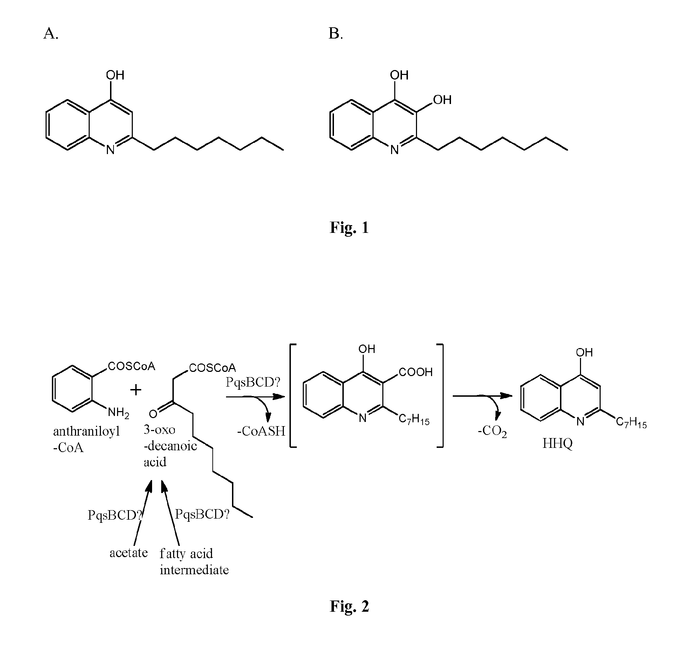

The opportunistic pathogen *Pseudomonas aeruginosa* is the causative agent of many nosocomial infections ranging from minor external infections to serious, life-threatening disorders, especially in immunocompromised and cystic fibrosis patients. The caused lung infections, with often lingering colonization and long-term lung damage, can be live threatening but in any case prolongate the patients convalescence thereby creating additional costs to the health system. The production of the bulk of the many different virulence factors excreted by *P. aeruginosa* is controlled by small molecule quorum sensing. In addition to the acyl homoserine lactone (AHL) group, which is widespread among many bacteria, *P. aeruginosa* and various related species use a vast variety of 2-hydroxy-4-alkyl quinolines (HAQs) which vary by different substituents on the quinoline ring as well as by secondary effects (e.g. antimicrobial) in order to regulate the expression of virulence factors. For regulation purposes most important are the *Pseudomonas* quinolone signal (PQS) and its direct precursor 2-hydroxy-4-heptyl quinoline (HHQ) (Figure 1).

Common to all these compounds is the quinoline core. Biosynthesis is thought to be mediated by the enzymes encoded by the *pqsABCDE* operon, using anthranilic acid as a precursor and condensing it with a β-ketocarboxylic acid yielding the quinoline core. *pqsA* encodes an anthranilate CoA ligase, while *pqsB,* C and D encode proteins similar to β-keto-acyl carrier protein synthases of type III. PqsE is not involved in the biosynthesis of HAQs. The PQS stimulus is mediated by the LysR-type transcriptional regulator PqsR (also known as MvfR). *pqsR* mutants of *P. aeruginosa* are described to exhibit decreased virulence in different host models (Cao H. et al., Proc. Natl. Acad. Sci. USA 2001; 98: 14613-14618). A very similar effect could be obtained by applying halogenated anthranilic acid analogues to *P. aeruginosa* cultures, which inhibited HAQ biosynthesis and thereby disrupted PqsR-dependent gene expression (Lesic B. et al., PLoS Pathog. 2007; 3:1229-1239). These results suggest that a targeted inhibition of HAQ biosynthesis in *P. aeruginosa* might be beneficial for therapy.

Although the genes essential for HHQ biosynthesis are known from inactivation experiments (pqsABCD), the biochemical process for HHQ biosynthesis has not been experimentally analysed until today. Figure 2 shows a scheme of the proposed biosynthesis of HHQ. It becomes obvious that the exact mode of action of PqsB, PqsC and PqsD is not known.

Serious *Pseudomonas aeruginosa* infections are difficult to treat. Usually, a combination of antibiotics is required because many strains, particularly those acquired in health care facilities, are resistant to many antibiotics. A series of pharmaceuticals is known for the treatment of bacterial and specifically of *P. aeruginosa* infections. However, side-effects and resistance are common problems associated with known anti-bacterial agents. This proves that new approaches and targets for anti-bacterial therapy are still needed.

So far, the exact role of the PqsD protein in the synthesis of HHQ and other HAQs has not been identified. Moreover, so far useful PqsD inhibitors have not been identified in the art, in particular for the purpose of treating, reducing or preventing a pathogenic infection. Targeting a pathogenic infection associated with quorum sensing of microoorganisms is still limited due to the absence of a suitable test system and screening assay.

Surprisingly, it has now been found that the PqsD protein is sufficient as the alone enzyme to catalyse the biosynthesis of HHQ which is an important regulator of quorum sensing from the substrates anthraniloyl-CoA and 3-oxodecanoic acid. Moreover, it has been found that the synthesis of HHQ can be performed in vitro. This new assay allows the in vitro-screening of inhibitors of HHQ biosynthesis.

Accordingly, the present invention provides in a first aspect a method of identifying a compound for treating, reducing or preventing a pathogenic infection caused by a microorganism, the method comprising the steps of:
a) providing a pqsD nucleic acid or a PqsD protein or a functional fragment or variant thereof,
b) contacting the pqsD nucleic acid or the PqsD protein or a functional fragment or variant thereof with a test compound,
c) determining the binding of the test compound to the pqsD nucleic acid or the PqsD protein or a functional fragment or variant thereof,
wherein the test compound is identified as a potential compound useful for treating, reducing or preventing a pathogenic infection, when the test compound binds to the pqsD nucleic acid or the PqsD protein or a functional fragment or variant thereof.

The present invention provides in a second aspect of the invention a method, wherein determining the binding of the test compound to the pqsD nucleic acid or the PqsD protein or a functional fragment or variant thereof comprises determining the activity of the PqsD protein or a functional fragment or variant thereof, wherein the test compound is identified as a potential compound useful for treating, reducing or preventing a pathogenic infection, when a reduction of the activity of the PqsD protein or a functional fragment or variant thereof in the test system relative to a control is detected.

The test system of the first and second aspect of the present invention may be used in order to elucidate mechanisms involved in the biosynthesis of HAQs, in particular of the biosynthesis of HHQ or of the biosynthesis of PQS. Particularly, the test system may be used to develop, identify and/or characterize compounds involved in the inhibition of HAQ biosynthesis, in particular of the biosynthesis of HHQ or of biosynthesis of PQS or associated with pathogenic infections or useful for treating, reducing or preventing a pathogenic infection. These compounds interact with the PqsD nucleic acid or the PqsD protein or a fragment or variant thereof, particularly inactivating the same. The identified compound may be an interesting therapeutic drug, which can be used in the treatment of a pathogenic infection.

The term "pathogenic infection caused by a microorganism" comprises an infection by an infectious microorganism which causes a disease in its host. An infection is the detrimental colonization of a host organism by a foreign species. In an infection, the infecting organism seeks to utilize the host's resources to multiply, usually at the expense of the host. The infecting organism interferes with the normal functioning of the host and can lead to chronic wounds, gangrene, loss of an infected limb, and even death. The host's response to infection is inflammation. In the present invention, the infectious microorganism comprises and uses quorum sensing for mediating infection by the production of virulence factors which are controlled by quorum sensing molecules. Microorganisms which use quorum sensing belong to the species of *Pseudomonas, Burkholderia, Alteromonas, Streptomyces, Vibrio* etc. Quorum sensing molecules are chemically diverse, ranging from peptides and N-acylhomoserine lactones (AHLs) to furanones and HAQs. Thus, in an embodiment of the present, the pathogenic infection to be treated, reduced or prevented by the compounds identified according to the method of the present invention is caused by a microorganism which comprises the quorum sensing molecules HAQs. In a preferred embodiment, the microorganism produces the quorum sensing molecule 4-hydroxy-2-alkyl quinoline wherein the alkyl chain is e.g. a pentyl, hexyl, heptyl, octyl, nonyl or undecyl group, preferably HHQ. In a further preferred embodiment, the HHQ molecule is produced by means of the PqsD protein. In a further preferred embodiment, the microorganism is a *Pseudomonas* or *Burkholderia* or *Alteromonas* species, further preferred a *Pseudomonas* species, in particular *Pseudomonas aeruginosa.*

*Pseudomonas aeruginosa* is member of the Gamma Proteobacteria class of Bacteria. It is a Gram-negative, aerobic rod belonging to the bacterial family Pseudomonadaceae. Since the revisionist taxonomy based on conserved macromolecules (e.g. 16S ribosomal RNA) the family includes only members of the genus Pseudomonas which are cleaved into eight groups. Pseudomonas aeruginosa is the type species of its group. which contains 12 other members. Like other members of the genus, *Pseudomonas aeruginosa* is a free-living bacterium, commonly found in soil and water. *Pseudomonas aeruginosa* has become increasingly recognized as an emerging opportunistic pathogen of clinical relevance. *Pseudomonas aeruginosa* is an opportunistic pathogen, meaning that it exploits some break in the host defenses to initiate an infection. In fact, *Pseudomonas aeruginosa* is the epitome of an opportunistic pathogen of humans. The bacterium almost never infects uncompromised tissues, yet there is hardly any tissue that it cannot infect if the tissue defenses are compromised in some manner. It causes urinary tract infections, respiratory system infections, dermatitis, soft tissue infections, bacteremia, bone and joint infections, gastrointestinal infections and a variety of systemic infections, particularly in patients with severe burns and in cancer and AIDS patients who are immunosuppressed. *Pseudomonas aeruginosa* infections generally persist despite the use of long-term antibiotic therapy. This is partially due to the fact that *P. aeruginosa* forms an antibioticresistant biofilm consisting of bacterial communities embedded in an exopolysaccharide matrix. Another problem caused by *Pseudomonas aeruginosa* is to be seen in association with the formation of biofilms on indwelling medical devices in the human body. Consequently, in a preferred embodiment, any of the diseases which are caused by *Pseudomonas aeruginosa* is treated, reduced or prevented in the sense of the present invention.

In one embodiment, the infectious microorganism as defined above forms a microbial biofilm within the infected individuum. The biofilm may develop in the organs which are colonized by the microorganism such as the lung. The biofilm may also develop on or within indwelling medical devices such as catheters, implantable drug delivery systems or prothesis or implants including contact lenses, central venous catheters and needleless connectors, endotracheal tubes, intrauterine devices, mechanical heart valves, pacemakers, peritoneal dialysis catheters, prosthetic joints, tympanostomy tubes, urinary catheters, and voice prostheses. A biofilm is an aggregate of microorganisms in which cells adhere to each other and/or to a surface. These adherent cells are frequently embedded within a self-produced matrix of extracellular polymeric substance (EPS). Biofilm EPS, which is also referred to as slime, is a polymeric conglomeration generally composed of extracellular DNA, proteins, and polysaccharides in various configurations. Biofilms may form on living or non-living surfaces, and represent a prevalent mode of microbial life in natural, industrial and hospital settings. Biofilms on indwelling medical devices may be composed of Gram-positive or Gram-negative bacteria or yeasts. Bacteria commonly isolated from these devices include the Gram-positive *Enterococcus faecalis, Staphylococcus aureus, Staphylococcus epidermidis,* and *Streptococcus viridans;* and the Gram-negative *Escherichia coli, Klebsiella pneumoniae, Proteus mirabilis,* and *Pseudomonas aeruginosa.* These organisms may originate from the skin of patients or health-care workers, tap water to which entry ports are exposed, or other sources in the environment. Biofilms may be composed of a single species or multiple species, depending on the device and its duration of use in the patient. A catheter is a tube that can be inserted into a body cavity, duct, or vessel. Catheters thereby allow drainage, injection of fluids, or access by surgical instruments. In most uses, a catheter is a thin, flexible tube ("soft" catheter), though in some uses, it is a larger, solid ("hard") catheter. A catheter left inside the body, either temporarily or permanently, may be referred to as an indwelling catheter. A permanently inserted catheter may be referred to as a permcath. Placement of a catheter into a particular part of the body may allow draining urine from the urinary bladder; drainage of urine from the kidney; drainage of fluid collections etc. An implantable drug delivery system is a device used to deliver a drug. Examples are medicament or drug pumps for the treatment of chronic pain or severe spasticity. Drug delivery systems are known in the art. Prosthesis is an artificial extension that replaces a missing body part. Prostheses are typically used to replace parts lost by injury or missing from birth or to supplement defective body parts. Prostheses outside the body are called exoprostheses, whereas prostheses inside the body are called endoprostheses or implants. Artificial hip or knee prostheses are classical endoprostheses, which may also designated as implants. There are known closed implants which are completely surrounded by body tissue in contrast to open implants which protrude out of the body. The most famous implant is the dental implant. Inside the body, artificial heart valves are in common use with artificial hearts and lungs and are under active technology development. Other medical devices and aids that can be considered prosthetics include artificial eyes, palatal obturator, gastric bands and dentures. Until today, biofilms on indwelling medical devices such prostheses and implants can be only removed by removing the device from the body, purifying the device, e.g., by thermal sterilization, and reintroducing the device into the body. Thus, in an embodiment of the invention a compound is identified for treating, reducing or preventing a pathogenic infection which is caused by an infectious microorganism which forms a biofilm either within an organ or on or within an indwelling device as referred to above and which comprises the PqsD protein. Preferably, the biofilm comprises a *Pseudomonas* species, in particular *Pseudomonas aeruginosa* as sole or additional component.

The term "treating, reducing or preventing a pathogenic infection" is used in its broadest sense and refers to both therapeutic treatment and prophylactic or preventative measures, wherein the object is to prevent or slow down the targeted pathologic condition or disorder. More specifically, treating, reducing or preventing is an intervention performed with the intention of preventing the development or altering the pathology of a pathogenic infection.

In a first step of the method of the first and second aspect of the present invention, a PqsD protein or a functional fragment or variant thereof is provided. In *Pseudomonas,* PqsD acts as a quinolone signal biosynthetic enzyme. *Pseudomonas* quinolone signal (PQS), 2-heptyl-3-hydroxy-4-quinolone, is an intercellular alkyl quinolone signaling molecule produced by the opportunistic pathogen *Pseudomonas aeruginosa.* PQS is synthesized from the tryptophan pathway intermediate, anthranilate. Anthranilate is converted to PQS by the enzymes encoded by the pqsABCDE operon which is responsible for generating multiple HAQs including 4-hydroxy-2-heptyl quinoline or termed otherwise 2-heptyl-4-quinolone (HHQ), the immediate PQS precursor, and pqsH. Exported HHQ is taken up by adjacent bacterial cells and converted into PQS by PqsH, a putative monooxygenase. In addition, PQS regulates its own production by driving the expression of pqsABCDE through a direct interaction with PqsR (MvfR). In the only biochemically characterized reaction, PqsA forms an activated anthraniloyl-CoA thioester that shuttles anthranilate to the PqsD active site where it is transferred to Cys112 of PqsD. A condensation then occurs between anthraniloyl-PqsD and malonyl-CoA or malonyl-ACP, a second PqsD substrate, forming 2,4-dihydroxyquinoline (DHQ). The role PqsD plays in the biosynthesis of other alkyl quinolones, such as PQS, however, remains unclear. Structural and biophysical characterization of PqsD that includes several crystal structures of the enzyme, including that of the PqsD-anthranilate covalent intermediate and the inactive Cys112Ala active site mutant in complex with anthranilate, has been reported. The characterization revealed that PqsD is structurally similar to the FabH and chalcone synthase families of fatty acid and polyketide synthases. The crystallographic asymmetric unit contains a PqsD dimer. The PqsD monomer is composed of two nearly identical approximately 170-residue alphabetaalphabetaalpha domains. The structures show that anthranilate-liganded Cys112 is positioned deep in the protein interior at the bottom of an approximately 15 Å long channel while a second anthraniloyl-CoA molecule is waiting in the cleft leading to the protein surface. Cys112, His257 and Asn287 form the FabH-like catalytic triad of PqsD. The C112A mutant is inactive, although it still reversibly binds anthraniloyl-CoA. Furthermore, it is reported that PqsD active site cavity seems inadequate for handling a substrate such as 3-oxodecanoyl-ACP which would be needed for the formation of HHQ. Therefore, they conclude that PqsD may not be involved in the biosynthesis of HHQ (Bera A.K. et al., Biochemistry 2009; 48: 8644-8655).

The PqsD sequence is available from the NCBI (National Centre for Biotechnology Information; National Library of Medicine 38A, Bethesda, MD20894, USA; www.ncbi.nih.gov) under the accession number ABJ10163.1 (SEQ ID NO: 1). The protein consists of 337 amino acids (including the initial methionine residue). The complete genome of *Pseudomonas aeruginosa* UCBPP-PA14 has been sequenced. The sequence is available from the NCBI under the accession number NC_008463: The pqsd gene is identified under the identification number GeneID: 4379983. *Pseudomonas aeruginosa* UCBPP-PA14 is a clinical isolate obtained from a burn patient that displays pathogenicity in a variety of genetically tractable model hosts and mice.

It has now surprisingly been found that the PqsD protein is suitable and sufficient as a sole enzyme to catalyse the reaction of anthraniloyl-CoA and 3-oxodecanoic acid to HHQ without the need for further enzymes such as PqsB or PqsC. In the sense of the present invention, the PqsD protein catalyses any reaction which it catalyses under natural conditions, preferably the conversion of anthraniloyl-CoA and 3-oxoalkanoic acid to 4-hydroxy-2-alkyl quinoline, more preferably of anthraniloyl-CoA and 3-oxo(C₄-C₂₃)alkanoic acid to 4-hydroxy-2-(C₁-C₂₀)alkyl quinoline and most preferably of anthraniloyl-CoA and 3-oxodecanoic acid to HHQ.

There exist at least five distinct classes of structurally related molecules designated in literature as HAQs, namely classes A to E. Their biosynthesis is inter alia regulated by the expression of the pqsABCDE operon. The HHQ molecule which is the most preferred molecule as produced by the method of the present invention belongs to the A series of the HAQ molecules. HAQ molecules are 4-hydroxy-2-alkyl quinolines. HHQ is characterized by carrying a heptyl group at position 2 of the quinoline ring structure. Other known HAQs of the A series differ from HHQ by a pentyl, hexyl, octyl, nonyl or undecyl group at position 2 of the quinoline ring structure. A proposed biochemical pathway of HAQs of the A series is presented in Figure 2. Thereby, it is proposed that anthraniloyl-CoA and 3-oxoalkanoic acid are converted to HAQ with alkyl chains of varying lengths at position 2 of the quinoline ring structure. This is exemplarily shown by the conversion of anthraniloyl-CoA and 3-oxodecanoic acid which results in HHQ with a C₇ alkyl chain at position 2 of the quinoline ring structure. Thus, in the context of the present invention, HAQ molecules are comprised which differ from HHQ only by the length of the alkyl chain at position 2 of the ring structure. The alkyl chain of the HAQ molecules thereby consists of 1 to 20 carbon atoms, more preferably of 5 to 11 carbon atoms and most preferably of 5, 6, 7, 8, 9 or 11 carbon atoms. The alkyl chains may be saturated or unsaturated, preferably they are saturated. The alkyl chains may be straight chain alkyl chains or branched chain alkyl chains. They may be of all stereoisomeric conformations of methyl, ethyl, propyl, butyl, pentyl, hexyl, heptyl, octyl, nonyl, decyl, undecyl, duode-cyl, tredecyl, quattordecyl, quindecyl, sesdecyl, septdecyl, octdecyl, nondecyl or eicosyl. They may be of all stereoisomeric conformations of ethylene, propylene, butylen, pentylen, hexylen, heptylen, octylen, nonylen, decylen, undecylen, duodecylen, tredecylen, quattordecylen, quindecylen, sesdecylen, septdecylen, octdecylen, non-decylen or eicosylen.

It should be noted that the conversion of anthraniloyl-CoA and 3-oxo(C₄-C₂₃)alkanoic acid results in an HAQ molecule wherein the length of the alkyl chain at position 2 of the quinoline ring structure is reduced by three carbon atoms with respect to the length of the substrate 3-oxo(C₄-C₂₃)alkanoic acid. Consequently, if 3-oxo(C₄-C₂₃)alkanoic acid is used, the product is 4-hydroxy-2-(C₁-C₂₀)alkyl quinoline. The reaction of anthraniloyl-CoA with 3-oxodecanoic acid results in the product HHQ.

Preferably, the PqsD protein as comprised by the present invention is derived from a *Pseudomonas* species, more preferably *Pseudomonas aeruginosa* and most preferably *Pseudomonas aeruginosa* UCBPP-PA14.

The term "PqsD protein" also encompasses naturally occurring PqsD proteins as they occur in the same *Pseudomonas* species from which the sequence of SEQ ID NO: 1 is derived (*Pseudomonas aeruginosa* UCBPP-PA14) and encompasses naturally occurring PqsD proteins as they occur in other *Pseudomonas* species and strains and *non-Pseudomonas* species such as *Burkholderia* or *Alteromonas* species where a quorum sensing mechanism is present and HAQs are produced requiring the presence of a PqsD protein or an ortholog or homolog thereof whereby this ortholog or homolog has the same function as the PqsD protein in *Pseudomonas aeruginosa.* Preferably, naturally occurring PqsD proteins or orthologs or homologs thereof differ from the sequence of SEQ ID NO: 1 e.g. by addition, deletion, substitution and/or insertion of amino acids and have a sequence identity with SEQ ID NO: 1 of more than 50%, of more than 60%, preferably of more than 80%, more preferably more than 85%, even more preferably more than 90%, even more preferably more than 95%, most preferably more than 97% and/or have an enzyme activity of more than 5%, more than 10%, more than 20%, more than 30%, more than 40%, more than 50%, more than 60%, more than 70%, more than 80%, more than 90%, more than 95%, more than 97% or more than 100 % of the enzymatic activity of the PqsD protein of SEQ ID NO: 1.

In the context of the present invention the PqsD protein as defined above or a naturally or non-naturally occurring variant thereof as comprised by the present invention is a functionally active protein in that the PqsD protein or variant maintains its biological function, i.e. its involvement in any reaction which it catalyses under natural conditions (in case of a non-natural variant, the biological function of the reference protein), preferably in the biosynthesis of quinolone signal, in particular in the biosynthesis of HAQs and more particularly in the synthesis of HHQ. Still more preferably, the PqsD protein or a variant thereof catalyses the conversion of anthraniloyl-CoA and 3-oxoalkanoic acid to 4-hydroxy-2-alkyl quinoline, still more preferably of anthraniloyl-CoA and 3-oxo(C₄-C₂₃)alkanoic acid to 4-hydroxy-2-(C₁-C₂₀)alkyl quinoline and most preferably of anthraniloyl-CoA and 3-oxodecanoic acid to HHQ.

Moreover, naturally occurring variants of the PqsD protein are also comprised by the term "PqsD protein". Such variants differ e.g. from the sequence of SEQ ID NO: 1 e.g. by addition, deletion, substitution and/or insertion of amino acids and have a sequence identity and protein activity as indicated above with respect to the naturally occurring PqsD protein.

Non-naturally occurring variants as comprised by the term "PqsD protein" may be obtained by a limited number of amino acid deletions, insertions and/or substitutions, particularly deletions, insertions and/or substitutions of at most 10, 9, 8, 7, 6, 5, 4, 3, 2 or 1 amino acid(s) thereby comprising a sequence identity or activity of the respective wild-type protein, e.g. with respect to SEQ ID NO: 1, as mentioned above.

The variant may be a modified PqsD protein or a modified PqsD protein variant which comprises a further component. Accordingly, the variant may be a molecule having a domain composed of a naturally occurring PqsD protein or a variant thereof as detailed herein and at least one further component. In one preferred embodiment the variant may be a fusion protein comprising (i) a PqsD protein or functionally active variant and (ii) a further protein component. For example, the protein may be coupled to a marker, such as a tag used for purification purposes (e.g. 6 His (or HexaHis) tag, Strep tag, HA tag, c-myc tag or glutathione S-transferase (GST) tag). If e.g. a highly purified PqsD protein or variant should be required, double or multiple markers (e.g. combinations of the above markers or tags) may be used. In this case the proteins are purified in two or more separation chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a hemagglutinin-pitope-tag), His-MBP (His-tag fused to a maltose-binding protein, FLAG-HA-tag (FLAG-tag fused to a hemagglutinin-epitope-tag), and the FLAG-Strep-tag. The marker could be used in order to detect the tagged protein, wherein specific antibodies could be used. Suitable antibodies include anti-HA (such as 12CA5 or 3F10), anti-6 His, anti-c-myc and anti-GST. Furthermore, the PqsD protein could be linked to a marker of a different category, such as a fluorescence marker such as green fluorescent protein, to a binding protein such as steptavidin, one or more small molecular dyes such as Cy dye, or a radioactive marker, which allows for the detection of PqsD. In a further embodiment, PqsD could be part of a fusion protein, wherein the second part could be used for detection, such as a protein component having enzymatic activity.

In another embodiment of the present invention, the PqsD variant could be a PqsD fragment, wherein the fragment is still functionally active. This may include PqsD proteins with short internal and/or C- and/or N-terminal deletions (e.g. deletions of at most 20, 19, 18, 17, 16, 15, 14, 13, 12, 11, 10, 9, 8, 7, 6 5, 4, 3, 2, or 1 amino acid). Additionally, the PqsD fragment may be further modified as detailed above for the PqsD protein.

Alternatively or additionally, the PqsD protein or variant thereof as described above may comprise one or more amino acid substitution(s). However, semi-conservative and especially conservative amino acid substitutions, wherein an amino acid is substituted with a chemically related amino acid are preferred. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues. Typical semi-conservative and conservative substitutions are:

| Amino acid | Conservative substitution | Semi-conservative substitution |
|---|---|---|
| A | G; S; T | N; V; C |
| C | A; V; L | M; I; F; G |
| D | E;N;Q | A;S;T;K;R;H |
| E | D; Q; N | A; S; T; K; R; H |
| F | W; Y; L; M; H | I; V; A |
| G | A | S; N; T; D; E; N; Q |
| H | Y; F; K; R | L;M;A |
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A;T;G;N | D; E; R; K |
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that glycines at sterically demanding positions should not be substituted and that P should not be introduced into parts of the protein which have an alpha-helical or a beta-sheet structure. The PqsD protein or fragment or variant thereof with substitution may be modified as detailed above for the PqsD protein or fragment or variant.

It is noted that the above modifications of the PqsD protein may be combined. The variant of the present invention may be e.g. a fragment of PqsD having a marker fused to it, or a PqsD protein fragment comprising one or more amino acid substitutions. Most preferably, the PqsD protein is a naturally occurring PqsD protein as detailed above, still more preferably, a naturally occurring *Pseudomonas* PqsD protein (e.g. SEQ ID NO: 1). In the following description of the invention all details given with respect to PqsD protein also relate to functionally active variants thereof, unless stated otherwise.

The required amounts of the PqsD protein may be provided by any means, e. g., by obtaining the PqsD protein from a natural source, by biotechnological methods well-known in the art and/or by synthetic methods. Here the term "providing" may be understood in the widest sense and may include but may not be limited to any biochemical or biotechnological means as known in the art that may be used to obtain the PqsD protein. Consequently, the PqsD protein may be obtained by extraction from a natural source such as a microorganism containing a PqsD protein, such as *Pseudomonas, Burkholderia* or *Alteromonas,* preferably a *Pseudomonas* species, more preferably *Pseudomonas aeruginosa* and most preferably *Pseudomonas aeruginosa* UCBPP-PA14. The skilled person will know methods of how to isolate and purify the protein. Cells naturally harbouring a PqsD protein may be disrupted by any means known in the art but not limited to sonication, hypotonic buffer, detergents, UltraTurrax, French press, freeze-thraw cycles, mechanical homogenization and scratching. The protein of interest may then be isolated by known methods.

Alternatively, the PqsD protein as used in the present invention may be a PqsD protein which is produced by recombinant methods, e.g. by cloning a pqsD nucleic acid encoding a PqsD protein or functional fragment or variant thereof and isolating the protein. The procedure of introducing a gene into a recipient cell is called transfection. Transfection with DNA yields stable as well as unstable (transient) cell lines. Transient cell lines reflect survival of the transfected DNA in extrachromosomal form; stable cell lines result from integration into the genome. The genes can be introduced into the cells by a variety of means known in the art and adapted to each cell type. The term "cell" refers to the cell in which the gene is expressed irrespective of whether it is a bacterial cell or a eukaryotic cell and of whether the cell naturally expresses the pqsD gene or not. Recombinant DNA cloning techniques well known in the art for introducing and expressing a nucleic acid molecule can be used to introduce and express the heterologous gene. Cells can be transfected using any appropriate means, including viral vectors, chemical transfectants, electroporation, calcium phosphate co-precipitation or direct diffusion of DNA. Vectors are agents that transport the heterologous gene into the cell and may include appropriate transcriptional and translational control signals such as a promoter. Vectors can be a plasmid, a virus or others as known in the art. The promoter can be inducible or constitutive, general or cell specific, nuclear or cytoplasmic specific. Selection of promoters, vectors and other elements is a matter of routine design. Many such elements are described in literature and are available through commercial suppliers. Usually, the method of transfer includes transfer of a selectable marker to the cells. In general, a cell line is transfected by any of the means mentioned above wherein the transgene is operatively linked to a selectable marker. Following transfection, cells are grown for an adapted period of time. Transfected cells exhibit resistance to the selection and are able to grow, whereas non-transfected cells die in general. Examples for selective markers include puromycin, zeocin, neomycin and hygromycin B which confer resistance to puromycin, zeocin, aminoglycoside G-418 and hygromycin, respectively. Examples of cells suitable in the context of the present invention include without limitation cells of *E. coli* strains, of yeast strains such as *Saccharomyces cerevisiae,* of the insect cell line *Lepidoptera* such as from *Spodoptera frugiperda,* of plant cells or of mammal cells such as L6 cells, 3T3 adipocytes, HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926.

An established or immortalised cell line has acquired the ability to proliferate indefinitely either through random mutation or deliberate modification, such as artificial expression of the telomerase gene. There are numerous well established cell lines representative of particular cell types and it is within the knowledge of the skilled person to select a suitable cell line. A cell line is a population of cells propagated in culture that are derived from, and therefore genetically identical to, a single common ancestor cell. Preferred cell lines are HEK 293 cells (primary human embryonic kidney), 3T3 cells (murine embryonic fibroblasts), CHO cells (Chinese hamster ovary), COS-7 cells (African green monkey cell line), HeLa cells (human epithelioid cervical carcinoma), JURKAT cells (human T-cell leukemia), BHK 21 cell (hamster normal kidney, fibroblast), and MCF-7 cells (human breast cancer).

The term "pqsD nucleic acid" encompasses nucleic acids coding for the above PqsD protein as well as naturally occurring and non-naturally occurring variants thereof (as defined herein). Preferably, the term relates to coding or non-coding regions of the pqsD gene, wherein these sections are of a relevant size in order to be specific for that gene. Examples of those regions are coding regions or regulatory elements such as a pqsD promoter. Most preferably, the term "pqsD nucleic acids" relates to pqsD gene, promoter, DNA, cDNA or mRNA.

The most preferred pqsD nucleic acids code for the naturally occurring PqsD protein as detailed above, still more preferably, a naturally occurring *Pseudomonas* PqsD protein (e.g. SEQ ID NO: 1). The nucleic acid may be any macromolecule composed of chains of monomeric nucleotides carrying genetic information or form structures within cells. The most common (and therefore preferred) nucleic acids are deoxyribonucleic acid (DNA) and ribonucleic acid (RNA).

The nucleic acid encoding the desired genetic information, preferably DNA, comprises the gene of interest, a promoter region, a start codon and a stop codon and possibly further regions which may be used for regulation of expression of the gene. The regulatory regions may be homologous or heterologous to the pqsD gene. The genetic information may be expressed permanently or under the control of a repressor and/or a promoter region. The obtained cells may be either used directly or used for tissue cultures or the cells may be harvested and samples comprising the PqsD protein are obtained by disrupting the cells.

Alternatively, DNA or RNA may be used either in cells or in cell-free expression systems such as, e.g., microarray systems in which the DNA or RNA is immobilized and is translated and/or transcribed by the addition of functional cell lysate, comprising the factors required for transcription and/or translation (enzymes, ribosomes, tRNA, amino acids, nucleotides, ATP etc.).

Alternatively, the PqsD protein may also be expressed in a tissue culture. The term "tissue culture" refers to method in which a group of cells forming a three dimensional network is cultivated. The tissue can be formed in culture by the cells themselves or may be formed by an extracellular matrix produced by the cells, the culture may be cultivated on a natural or an artificial extracellular matrix (e.g. collagen, elastin, polystyrene, nylon, polylysine) or the tissue may be obtained from an animal including human. The tissue culture may be cultivated in culture medium and at suitable temperature. The culture medium may contain nutrients (e.g. sugars, salts, amino acids, and lipids), a buffer system (often comprising one or more chemical buffer substances such as phosphates, hydrogen phosphates and/or Tris with low or no toxicity and/or carbon dioxide (CO₂) gassing) and/or optionally one or more antibiotics. In order to provide sufficient amounts of nutrients and/or to remove metabolites the medium may be changed when required. Optionally, the tissue culture may be perfused with medium. The perfusion may be a permanent or pulsed perfusion.

Artificial nucleic acids include peptide nucleic acid (PNA), morpholino and locked nucleic acid (LNA), as well as glycol nucleic acid (GNA) and threose nucleic acid (TNA). Each of these is distinguished from naturally-occurring DNA or RNA by changes to the backbone of the molecule.

When conducting the method, the provided pqsD nucleic acid or PqsD protein is contacted with a compound as comprised by the second step of the first and second aspect of the present invention. In the context of the present invention, the term "contacting" may be understood in the widest sense as any means that allow an interaction of the PqsD protein with one or more compounds that may be tested. Preferably, this is achieved by mixing one or more solutions containing the PqsD protein and one or more compounds in a buffer solution suitable for the enzymatic activity of the PqsD protein. The mixing may preferably be accomplished by pipetting, more preferably by mixing two or more solutions of which one contains the PqsD protein and another one contains the compound. In a preferred embodiment, the contacting may be accomplished by automated pipetting.

Throughout the invention, the term "test compound" or "compound" may refer to any test substance of any chemical nature. It may refer to a small molecule with a molecular weight of less than 500 daltons in size or a salt thereof, a high-molecular weight compound such as a protein, a polypeptide, deoxyribonucleic acid (DNA), ribonucleic acid (RNA), a synthetic polymer, an antibody and/or antibody derivative (e.g. Fab fragments, single chain antibodies, diabodies, triabodies, flexibodies, tandabs). The compound may be charged or uncharged. Furthermore, the compound may be hydrophilic, hydrophobic or amphiphilic. The compound may be one or more inorganic salts, one or more salt ions, one or more metals, one or more complexes of one or more inorganic salts or ions and/or a complex of one or more metals, preferably complexed with one or more organic molecules. Alternatively, the compound may be a gas. Preferably, the compound may be soluble in an aqueous buffer or an aqueous solvent. The compound may already have been used as a drug or a medicament. Alternatively, the compound may also be a chemical substance without known therapeutic use or even a substance with unknown chemical properties used in the assay. Furthermore, the term "test compound" or "compound" may also include a substance that may serve as a precursor of a molecule that is used in the method. In particular, this may apply for instable substances that require the synthesis immediately before measuring. Additionally, this may apply for prodrugs as often used in pharmacology, which are molecules that have to be metabolized before displaying their action (e.g. acetylated, lapidated or oxidized precursors of the active compounds).

Alternatively or additionally, the compound may be conjugated or bound to one or more other molecules, such as small molecules, peptides, proteins, polysaccharides or synthetical polymers. Preferably, it may be conjugated to one or more small molecule dyes (e.g. fluorescent dyes such as a Cy dye (e.g. Cy3, Cy5, Cy5), an Alexa dye (e.g. Alexa488, Alexa 546, Alexa 647), S dyes (e.g. S0387), fluorescein and functional derivatives thereof (e.g. FITC), rhodamine, HOECHST dye, etc. or UV/Vis dyes), one or more quantumdots, one or more binding moieties (e.g., biotin, glycocorticoids or moieties comprising, e.g., one or more active esters, isothiocyanates, maleimids or combinations thereof), one or more soluble and/or insoluble polymers (e.g. polyethylene glycol (PEG), hydroxypropyl methacrylate (HPMA)), one or more spin labels, one or more and/or micro- or nanobeads (e.g. functionalized silica beads, sugar-based beads, polymersomes). Furthermore, the compound may be radioactively labeled, e.g., with ³H, ³²P, ³⁵5, ¹⁴C or lanthonoids suitable for scintillation assays or computer tomography (CT) or with labels suitable for positron emission tomography (PET).

As used herein, the term "method" or "assay" may be understood in the widest sense as an experimental activity or procedure. It will be understood as all means that may be used to identify a compound with certain properties or to characterize a certain compound. It may include the testing of one or more samples as well as the screening of libraries containing numerous samples.

The term "identifying a compound" means in the widest sense to find a compound showing an effect on the enzymatic activity of the PqsD protein when contacted therewith, in particular a compound showing an inhibitory effect on the enzymatic activity of the PqsD protein when contacted therewith. A compound showing such an inhibitory effect is found to have an effect of treating, reducing or preventing a pathogenic infection.

The term "inhibitor" may be understood interchangeable with "antagonist", "inactivator", "inactivating agent", "interacting drug" "compound with inhibitory effect" and "inactivating compound" and other designations for a substance that inactivates enzymatic activity. Inhibitors are molecules that bind to enzymes and decrease their activity. Since blocking an enzyme's activity can correct a metabolic imbalance, many drugs are enzyme inhibitors. Not all molecules that bind to enzymes are inhibitors; enzyme activators bind to enzymes and increase their enzymatic activity. The binding of an inhibitor can stop a binding partner from interacting with the biomolecule and/or hinder the biomolecule from being active or activated. Inhibitor binding is either reversible or irreversible. Irreversible inhibitors usually react with the biomolecule and change it chemically. These inhibitors may e.g. modify key amino acid residues needed for the activity. In contrast, reversible inhibitors bind non-covalently and different types of inhibition are produced depending on whether these inhibitors bind the biomolecule. If the compound has a specific and significant reducing effect on the PqsD protein, the compound is identified as an inhibitor of the PqsD protein and, therefore, as a compound having an effect of treating, reducing or preventing a pathogenic infection. An inhibitor of the PqsD protein in the context of the present invention means a compound decreasing PqsD enzyme activity in comparison to a control. The person skilled in the art knows statistical procedures to assess whether two values are significantly different from each other such as Student's t-test or chi-square tests.

In a third step of the first and second aspect of the present invention, the effect of the test compound on the PqsD protein is detected. The effect is determined as the binding of the test compound to a pqsD nucleic acid or PqsD protein as defined above. In a preferred embodiment, the activity of the PqsD protein is determined as a consequence of the binding of the test compound to the PqsD protein. In the following, a series of different detection systems will be described in more detail. However, it should be understood that these are exemplary and other test systems and methods may be also appropriate.

If the test compound has a specific and significant effect on the PqsD protein, the test compound is identified as compound useful for treating, reducing or preventing a pathogenic infection. For this, the effect of the test compound is compared to a control, particularly a negative control.

Controls are a part of the test methods, since they can eliminate or minimize unintended influences (such as background signals). Controlled experiments are used to investigate the effect of a variable on a particular system. In a controlled experiment one set of samples has been (or is believed to be) modified and the other set of samples is either expected to show no change (negative control) or expected to show a definite change (positive control). The control can be determined in one test run together with the test substance. It may be determined before or after determining the effect of the test compound or it may be a known value.

In the context of the present invention, the test compound has an effect in comparison to a control, if the PqsD protein contacted with the test compound produces a PqsD activity significantly lower than that of a control (e.g. test system not contacted with the test compound). Additionally, the background, the conversion of the substrate independent of PqsD activity, may be measured by means of a sample containing the substrate in the respective buffer lacking the enzyme. In this background sample, there will be no or nearly no conversion of the substrate. The person skilled in the art knows statistical procedures to assess whether two values are significantly different from each other such as Student's t-test or chi-square tests. Furthermore, the skilled person knows how to select a suitable control. In a preferred embodiment, the PqsD activity is altered by the test compound by at least 10 %, preferably at least 25 %, more preferably at least 50 %, still more preferably at least 75 % and most preferably at least 90 % of the control.

For the method of the invention any suitable method of detecting may be used. Suitable methods may be chosen depending on the characteristics of the test system and compounds to be tested. The method may be a heterogeneous or homogeneous assay. As used herein, a heterogeneous assay is an assay which includes one or more washing steps, whereas in a homogeneous assay such washing steps are not necessary. The reagents and compounds are only mixed and measured.

The test method may be either a continuous assay or a discontinuous assay. Continuous assays give the rate of reaction with no further work necessary. There are many different types of continuous assays. In spectrophotometer assays, the course of the reaction is followed by measuring a change in absorbance. Fluorescence is when a molecule emits light of one wavelength after absorbing light of a different wavelength. Fluorometric assays use a difference in the fluorescence of substrate from product to measure the enzyme reaction. These assays are in general much more sensitive than spectrophotometric assays, but can suffer from interference caused by impurities and the instability of many fluorescent compounds when exposed to light. Calorimetry is the measurement of the heat released or absorbed by chemical reactions. These assays are very general, since many reactions involve some change in heat and with use of a microcalorimeter, not much enzyme or substrate is required. These assays can be used to measure reactions that are impossible to assay in any other way. Chemiluminescence is the emission of light by a chemical reaction. Some enzyme reactions produce light and this can be measured to detect product formation. These types of assay can be extremely sensitive, since the light produced can be captured by photographic film over days or weeks, but can be hard to quantify, because not all the light released by a reaction will be detected. Static Light Scattering measures the product of weight-averaged molar mass and concentration of macromolecules in solution. Given a fixed total concentration of one or more species over the measurement time, the scattering signal is a direct measure of the weight-averaged molar mass of the solution, which will vary as complexes form or dissociate. Hence the measurement quantifies the stoichiometry of the complexes as well as kinetics. Light scattering assays of protein kinetics is a very general technique that does not require an enzyme.

Discontinuous assays are when samples are taken from an enzyme reaction at intervals and the amount of product production or substrate consumption is measured in these samples. Radiometric assays measure the incorporation of radioactivity into substrates or its release from substrates. The radioactive isotopes most frequently used in these assays are ¹⁴C, ³²P, ³⁵S and ¹²⁵I. Since radioactive isotopes can allow the specific labeling of a single atom of a substrate, these assays are both extremely sensitive and specific. They are frequently used in biochemistry and are often the only way of measuring a specific reaction in crude extracts. Chromatographic assays measure product formation by separating the reaction mixture into its components by chromatography. This is usually done by high-performance liquid chromatography (HPLC), but can also use the simpler technique of thin layer chromatography. Although this approach can need a lot of material, its sensitivity can be increased by labeling the substrates/products with a radioactive or fluorescent tag.

Evidently, the enzyme activity is influenced by a series of factors including the amount of enzyme, the activation status of the enzyme, the presence of cofactors such as a co-activator or a co-repressor, the presence of activators and inhibitors and the ambient condition such as salt concentration, temperature, pH etc. Usually, the enzyme activity is measured at standard laboratory conditions and may be adapted to the optimum of the test system in question. Accordingly, the test system may be used in order to detect or identify molecules changing the activation status of the enzyme such as activators and inhibitors, which might be useful therapeutics.

The effect, e.g. binding, of the test compound and the influence on the PqsD protein can be detected indirectly. In an especially preferred embodiment, the binding of the test compound is measured by the change of the activity of the PqsD. In order to obtain the desired enzyme activity, the measurements may be carried out in a molecular environment suitable for the activity of the PqsD protein. Preferably, the method is carried out in an aqueous buffer of neutral to mild basic pH, more preferably in a buffer with a pH around pH 8, even more preferably in a buffer of pH 8 comprising Tris-HCl, HEPES, BICINE, MES, triethanolamine or phosphate buffer. Preferably, the PqsD protein maintains its activity in the used buffer and preferably compound or enzyme precipitation is prohibited. Optionally, the samples may be incubated at a temperature suitable for the enzyme activity, preferably at temperatures between 0 and 50°C, more preferably at temperatures between 10 and 40 °C, even more preferably at temperatures between 20 and 40°C, most preferably at 37°C. The applied methods are described in more detail in the examplary section. However, it should be understood that these are exemplary and other methods may be appropriate as well. It might be obvious to a person skilled in the art that the enzyme has to be diluted to the desired concentration and that this concentration may vary for different batches due to the varying enzymatic activity per amount of enzyme.

In a preferred embodiment, the activity of the PqsD protein is determined by measuring the conversion of a substrate of the PqsD protein into a product. The conversion of a substrate relates to the enzymatic, thus, to the PqsD protein-catalyzed alteration or metabolism of a chemical compound. Throughout the invention, the term "substrate" relates to any chemical substance that may be metabolized by the PqsD protein into a product. In the present method, detectable natural substrates of the PqsD protein as they occur in organisms which harbour a PqsD protein as defined above may be used to enable the detection of the enzymatic activity of the PqsD protein. Moreover, detectable surrogates of such natural substrates may be used to enable the detection of the enzymatic activity of the PqsD protein or variant thereof. Preferably, these natural substrates or surrogates and the products resulting therefrom may be detectable by physicochemical means, more preferred by HPLC methods as detailed below. The term "surrogate" may be understood in the widest sense as a molecule that can be metabolized and/or enzymatically converted instead of the natural substrate(s).

For the biosynthesis of HAQ, two substrates are converted by PqsD. Therefore, in a preferred embodiment the first substrate is anthranilic acid, preferably a derivative of anthranilic acid such as anthraniloyl-CoA. The second substrate is preferably 3-oxoalkanoic acid, more preferably 3-oxo(C₄-C₂₃)alkanoic acid and most preferably 3-oxodecanoic acid. In the most preferred embodiment, the first substrate is anthraniloyl-CoA and the second substrate is 3-oxodecanoic acid. Also included are embodiments wherein the substrate 3-oxoalkanoic acid, preferably 3-oxo(C₄-C₂₃)alkanoic acid and more preferably 3-oxodecanoic acid may be linked to a substance activating the substance and thereby supporting the conversion of substrate to product. The linkage may be a covalent linkage. Preferred substances are Coenzyme A or ACP (acyl carrier protein) the binding of which results in the corresponding thioester of 3-oxoalkanoic acid, preferably 3-oxo(C₄-C₂₃)alkanoic acid and more preferably 3-oxodecanoic acid. In the context of the present invention, 3-oxoalkanoic acid, preferably 3-oxo(C₄-C₂₃)alkanoic acid and more preferably 3-oxodecanoic acid need not to be derivatised as indicated above to be useful in the conversion reaction with anthraniloyl-CoA to HAQ, but the reaction proceeds, if only anthranilic acid is present in the reaction mixture in an activated form, i.e. as anthraniloyl-CoA. Vice versa, Coenzyme A or ACP derivatised 3-oxoalkanoic acid, preferably 3-oxo(C₄-C₂₃)alkanoic acid and more preferably 3-oxodecanoic acid may be used in the enzymatic assay in combination with anthraniloyl-CoA or with anthranilic acid without Coenzme A. At least one of the two substrates should be present in an activated for, i.e anthranilic acid as anthraniloyl-CoA or the HAQ molecule as HAQ-COA or HAQ-ACP.

Anthraniloyl-CoA is one of the substrates of PqsD which is converted together with 3-oxoalkanoic acid, preferably with 3-oxo(C₄-C₂₃)alkanoic acid and most preferably 3-oxodecanoic acid to 4-hydroxy-2-alkyl quinoline, preferably to 4-hydroxy-2-(C₁-C₂₀)alkyl quinoline and more preferably HHQ, respectively. Anthraniloyl-CoA is predicted to react with β-keto fatty acid via a head-to-head condensation in a series of steps requiring PsqB, PsqC and PsqD to form HAQ or preferably HHQ. In *Pseudomonas,* anthraniloyl-CoA is formed from anthranilic acid by PqsA which is an anthraniloyl-CoA synthetase. In the context of the present invention, any method for producing anthraniloyl-CoA may be used. The skilled person will be aware of methods for producing anthraniloyl-CoA. In one embodiment of the method of the present invention, anthraniloyl-CoA is formed from anthranilic acid. Anthranilic acid is e.g. available from commercial sources. The production of anthraniloyl-CoA may occur by enzymatic catalysis by any enzyme which converts anthranilic acid into anthraniloyl-CoA. For example, PqsA may be used to convert anthranilic acid into anthraniloyl-CoA. PqsA is a known anthranilate-coenzyme A ligase. PqsA protein is homologous to a variety of acyl coenzyme A (acyl-CoA) ligases and the obvious role of PqsA is to convert anthranilate into the CoA thioester. This product is the precursor for the condensation step that gives final 4-qinolone. The enzyme is available from the NCBI under the accession number ABJ10160.1 (SEQ ID NO: 2). The complete genome of *Pseudomonas aeruginosa* UCBPP-PA14 has been sequenced. The sequence is available from the NCBI under the accession number NC_008463: The pqsA gene is identified under the identification number GeneID: 4379980.

The term "PqsA" or "PqsA protein" as comprised by the present invention encompasses naturally occurring PqsA proteins as they occur in the same species from which the sequence of SEQ ID NO: 2 is derived (*Pseudomonas aeruginosa* UCBPP-PA14) or encompasses naturally occurring PqsA proteins as they occur in other *Pseudomonas* species and strains and *non-Pseudomonas* species, provided the PqsA protein has the same activity as the protein of SEQ ID NO: 2, namely it catalyses the conversion of anthranilic acid into anthraniloyl-CoA. Also comprised are naturally or non-naturally occurring variants which are defined as mentioned above with respect to the PqsD protein.

In a more preferred embodiment, 2-aminobenzoate-CoA ligase is used which converts anthranilic acid into anthraniloyl-CoA. In a still more preferred embodiment, 2-aminobenzoate-CoA ligase is from *Azoarcus evansii.* The enzyme is available from the NCBI under the accession number AAL02077.1 (SEQ ID NO: 3) with the corresponding nucleic acid sequence as available under the accession number AF320254.1.

The term "2-aminobenzoate-CoA ligase" encompasses naturally occurring 2-aminobenzoate-CoA ligases as they occur in the same species from which the sequence of SEQ ID NO: 3 is derived (*Azoarcus evansii*) or encompasses naturally occurring 2-aminobenzoate-CoA ligases as they occur in other *Azoarcus* species and strains and *non-Azoarcus* species, provided the enzyme has the same activity as the protein of SEQ ID NO:3, namely it catalyses the conversion of anthranilic acid to anthraniloyl-CoA. Also comprised are naturally and non-naturally occurring variants which are defined as mentioned above with respect to the PqsD protein.

PqsA protein and 2-aminobenzoate-CoA ligase can be prepared as described above with respect to the PqsD protein.

The second substrate of the PqsD protein is 3-oxoalkanoic acid, more preferably 3-oxo(C₄-C₂₃)alkanoic acid and most preferably 3-oxodecanoic acid. The skilled person will be able to produce 3-oxoalkanoic acid, more preferably 3-oxo(C₄-C₂₃)alkanoic acid and most preferably 3-oxodecanoic acid by methods known in the art. In the experimental procedures, the synthesis of 3-oxo-decanoic acid is exemplarily disclosed. The skilled person will be aware of procedures to synthesize 3-oxoalkanoic acids, e.g. 3-oxo(C₄-C₂₃)alkanoic acid. Moreover, the substances are commercially available.

In the context of the present invention the PqsD protein or a functionally active fragment or variant thereof and one or more of the test compound(s) are contacted with the substrates as indicated above under conditions which are suitable for the reaction to proceed. Such conditions are as indicated above. The compound(s) may be added to the reaction mixture before the experiment begins or while the reaction proceeds. In a preferred embodiment anthraniloyl-CoA, the PqsD protein and the test compound are contacted for a time and under conditions suitable for the components to become temperature equilibrated. The test system is then contacted and the reaction started by addition of 3-oxoalkanoic acid, more preferably 3-oxo(C₄-C₂₃)alkanoic acid and most preferably 3-oxodecanoic acid for a time and under conditions suitable for having an effect on the test system and detecting the same. Suitable conditions for the equilibration step and the reaction step include appropriate temperature, time and solution to allow equilibration of substrates and enzyme and conversion of substrates to product and to avoid e.g. denaturation of proteins involved. Suitable conditions will depend from the particular test system chosen and the skilled person will be able to select the same based on his general knowledge. Equilibration and incubation steps can vary from about 5 seconds to several hours, preferably from about 1 minute to about 10 minutes, most preferably 2 minutes. However, the equilibration and incubation time will depend upon the assay format, marker, volume of solution, concentrations and the like.

For measuring the change of PqsD activity, the effect upstream or downstream the PqsD protein can be detected. For example, the effect on substrate conversion or product formation can be determined. In one embodiment, the change of amount of substrate or amount of product is detected.

In the context of the present invention an enzymatic conversion of substrate, being preferably anthraniloyl-CoA and 3-oxoalkanoic acid, preferably 3-oxo(C₄-C₂₃)alkanoic acid and more preferably 3-oxodecanoic acid, to the respective product occurs by use of the PqsD protein or variant thereof. During the course of reaction substrate is consumed and the concentration decreases whereas product is formed and the concentration increases. For determining of whether a test compound inhibits the production of a product, both substrate components and the PqsD protein are assigned to fixed values. The test compound, the substrate component anthraniloyl-CoA and the enzyme are mixed and temperature equilibrated. The reaction is started by the addition of the second substrate being 3-oxoalkanoic acid, preferably 3-oxo(C₄-C₂₃)alkanoic acid and more preferably 3-oxodecanoic acid, and incubated for a fixed period. The decrease of substrate concentration or the increase of product concentration in a fixed time interval can then be determined by methods known to those skilled in the art. Comparison with a control reaction, preferably a control reaction wherein no inhibitor is added, indicates of whether the test substance is capable of inhibiting the conversion of the substrates into product and thus of inhibiting the activity of the PqsD protein. For example, if the decrease of the substrate molecules is less than within the control reaction or if the increase of the product molecule is less than in the control reaction, then inhibition of the conversion occurs and the test compound is identified as an inhibitor useful in the treatment, reduction or prevention of a pathogenic infection. In a preferred embodiment, the decrease of substrate or increase of product is altered by the test compound by at least 10 %, preferably at least 25 %, more preferably at least 50 %, still more preferably at least 75 % and most preferably at least 90 % as compared to the control.

As in the context of the present invention the effect of treating, reducing or preventing a pathogenic infection is preferably measured as the inhibitory impact of the compound on the enzymatic activity of the PqsD protein, the threshold may preferably be given as a certain IC₅₀ value. As commonly known in the art, the IC₅₀ value refers to the compound concentration required for the half maximal inhibition of the enzymatic activity. Evidently, the IC₅₀ value is a relative value dependent on a series of factors including the amount of enzyme, the activation status of the enzyme, the presence of cofactors such as a co-activator or a co-repressor, the presence of activators and inhibitors and the ambient condition such as salt concentration, temperature, pH etc. In the context of the present invention, a compound may preferably be identified as a compound useful for treating, reducing or preventing a pathogenic infection when the IC₅₀ value is below 50 µM, below 20 µM, below 10 µM, below 5 µM, below 4 µM, below 3 µM, below 2 µM, below 1 µM, below 0.5 µM, below 0.4 µM, below 0.3 µM, below 0.2 µM, below 0.1 µM, below 0.05 µM, below 0.02 µM or below 0.01 µM.

A variety of assays is known in the art by which the skilled person may determine the concentration of a compound and thus measure of whether the concentration of a substrate or product is changed versus a control reaction. Explicitly mentioned are radiometric assays which measure the incorporation of radioactivity into substances or its release from substances. The radioactive isotopes most frequently used in these assays are ¹⁴C, ³²P, ³⁵S and ¹²⁵I. Chromatographic assays measure product formation by separating the reaction mixture into its components by chromatography. This is usually done by high-performance liquid chromatography (HPLC), but can also use the simpler technique of thin layer chromatography. Although this approach can need a lot of material, its sensitivity can be increased by labeling the substrates/products with a radioactive or fluorescent tag. A preferred method is quantification is by HPLC/MS.

Liquid chromatography-mass spectrometry (LC-MS, or alternatively HPLC-MS) is an analytical chemistry technique that combines the physical separation capabilities of liquid chromatography (or HPLC) with the mass analysis capabilities of mass spectrometry. LC-MS is a powerful technique used for many applications which has very high sensitivity and specificity. Generally its application is oriented towards the specific detection and potential identification of chemicals in the presence of other chemicals (in a complex mixture).

The term "mass spectrometry" refers to the use of a ionization source to generate gas phase ions from a sample on a surface and detecting the gas phase ions with a mass spectrometer. The term "laser desorption mass spectrometry" refers to the use of a laser as a ionization source to generate gas phase ions from a sample on a surface and detecting the gas phase ions with a mass spectrometer. A preferred method of mass spectrometry for biomolecules such as acylated acyl acceptor is matrix-assisted laser desorption/ionization mass spectrometry or MALDI. In MALDI, the analyte is typically mixed with a matrix material that, upon drying, co-crystallizes with the analyte. The matrix material absorbs energy from the energy source which otherwise would fragment the labile biomolecules or analytes. Another preferred method is surface-enhanced laser desorption/ionization mass spectrometry or SELDI. In SELDI, the surface on which the analyte is applied plays an active role in the analyte capture and/or desorption. In the context of the invention the sample comprises a biological sample that may have undergone chromatographic or other chemical processing and a suitable matrix substrate. In mass spectrometry the "apparent molecular mass" refers to the molecular mass (in Daltons)-to-charge value, m/z, of the detected ions. How the apparent molecular mass is derived is dependent upon the type of mass spectrometer used. With a time-of-flight mass spectrometer, the apparent molecular mass is a function of the time from ionization to detection. The term "signal" refers to any response generated by a biomolecule under investigation. For example, the term signal refers to the response generated by a biomolecule hitting the detector of a mass spectrometer. The signal intensity correlates with the amount or concentration of the biomolecule. The signal is defined by two values: an apparent molecular mass value and an intensity value generated as described. The mass value is an elemental characteristic of the biomolecule, whereas the intensity value accords to a certain amount or concentration of the biomolecule with the corresponding apparent molecular mass value. Thus, the "signal" always refers to the properties of the biomolecule.

In combination with HPLC the analyte is ionized by diverse API techniques such as ESI (elektrospray ionization) or APCl (atmospheric pressure chemical ionization). In the analysator, the ions are separated according to their mass-to-charge m/z ratio. In a particularly preferred embodiment substrate or product concentration such as the concentration of the substrate anthraniloyl-CoA and/or 3-oxoalkanoic acid, preferably 3-oxo(C₄-C₂₃)alkanoic acid or more preferably 3-oxodecanoic acid or of the product 4-hydroxy-2-alkyl quinoline, preferably 4-hydroxy-2-(C₁-C₂₀)alkyl quinoline or more preferably HHQ is determined by HPLC/MS. In a more preferred embodiment formation of the product such as 4-hydroxy-2-alkyl quinolines, preferably 4-hydroxy-2-(C₁-C₂₀)alkyl quinoline or more preferably HHQ is quantified by HPLC/MS. Fletcher et al., Nature Protocols 2:1254-1262 (2007) describe biosensor-based assays for determining PQS, HHQ and related 2-alkyl-4-quinolone molecules whereby the HAQs are detected via bioluminescence of a specifically constructed reporter strain. These assays are time-consuming and labour-intensive, have only low possibility for automatisation and involve the contact with potentially pathogenic agents. Detection of compounds (substrates and products) by HPLC-MS in connection with the in vitro conversion of the substrates such as anthraniloyl-CoA and 3-oxoalkanoicc acid, preferably 3-oxo(C₄-C₂₃)alkanoic acid or more preferably 3-oxodecanoic acid to the product such as 4-hydroxy-2-alkyl quinolines, preferably 4-hydroxy-2-(C₁₋C₂₀)alkyl quinoline or more preferably HHQ offers the possibility of rapid performance as well as the potential for automatisation and safe handling. This opens the possibility, to screen for inhibitors in already existing substance libraries. In view of the advantages of the HPLC-MS system, this system is the preferred system for quantification of substrates and/or products and preferably of products in the present invention.

The most preferred embodiment of the present invention is a method wherein anthraniloyl-CoA and 3-oxodecanoic acid are converted by PqsD of SEQ ID NO: 1 in the presence of a test compound and enzyme activity is measured by measuring the amount of HHQ by HPLC-MS versus a control reaction containing no test compound. The reaction is carried out in vitro in a suitable cell-free reaction solution.

Alternatively, PqsD activity may be measured by measuring HAQ antimicrobial activity.

In another aspect of the present invention, the binding and influence of the test compound on the PqsD protein may be directly determined by determining an interaction with or binding to the PqsD protein. Accordingly, the interaction/binding of a test compound to the PqsD protein can be determined by detecting the complex of the PqsD protein and the test compound. Such an interaction may enhance or may reduce the activity of the PqsD protein. In one embodiment, determining the ability of the test compound to bind to or interact with the PqsD protein can be accomplished by determining the activity of the PqsD protein as outlined above. In the context of the present invention, the test compound is suitable for treating, reducing or preventing a pathogenic infection if it reduces the activity of the PqsD protein. Suitable methods of detecting complexes of two or more components are detailed below.

Many known methods for detection that are designed to measure the presence or quantity of specific proteins or other nucleic acids depend on the use of tags, markers or labels. A component of the test system or the test compound may be labeled in a variety of ways to allow sufficient detection or purification. In one preferred embodiment a detectable marker is used in order to detect an effect on the test system. For this, the PqsD protein, the test compound and/or a further component of the test system may be labeled with at least one detectable marker.

Common labeling methods may be used for labeling one or more functional groups of the component. For a protein, these could be for example the primary amino groups, present at the N-terminal of each polypeptide chain and the side chain of lysine residues; sulphhydryl groups, present on cysteine residues made available by treating disulphide bonds with reducing agent or by modifying lysine residues with a reagent such as succinimidyl-S-acetylthioacetate (SATA); or carbohydrate groups, usually present in the Fc region of antibodies, which may be oxidized to create active aldehydes for coupling. The component or compound may be labeled with a series of different agents, such as biotin (for avidine-biotin chemistry), enzymes, activated fluorescent dyes for labeling amines, sulphhydryls or other functional groups with e.g. FITC, fluorescein, rhodamine, Cy dyes or Alexa fluos. Radioactive label such as ³H, ³²P, ³⁵S, ¹²⁵I or ¹⁴C as well as common enzyme labels including penicillinase, horseradish peroxidase and alkaline phosphatase may be used as well.

The use of target-specific probes that are detectable via those chemical tags, markers or labels is envisaged by the present invention. Antibodies are the most common type of probe; their binding affinities for particular antigens enable those targets to be "found" and detected in a complex sample. However, antibodies are themselves proteins, and they are not specifically detectable in an assay system unless they are labeled for visualization or secondarily probed with another molecule that is labeled.

A marker (or tag or label) is any kind of substance which is able to indicate the presence of another substance or complex of substances. The marker can be a substance that is linked to or introduced in the substance to be detected. Detectable markers are used in molecular biology and biotechnology to detect e.g. a protein, a product of an enzymatic reaction, a second messenger, DNA, interactions of molecules etc. Examples of suitable marker or labels include a fluorophore, a chromophore, a radiolabel, a metal colloid, an enzyme, or a chemiluminescent or bioluminescent molecule. Examples of fluorophores include fluorescein, rhodamine, and sulfoindocyanine dye Cy5. Examples of radiolabels include ³H, ¹⁴C, ³²P, ³³P, ³⁵S, ^{99m}Tc or ¹²⁵I. Examples of enzymes include horseradish peroxidase, alkaline phosphatase, glucose oxidase, and urease.

Different types of chemical labels or tags can be conjugated to secondary or primary antibodies and other molecules to facilitate their visualization (i.e., detection and measurement) by various methods. Radioisotopes were used extensively in the past, but they are expensive, have a short shelf-life, offer no improvement in signal:noise ratio and require special handling and disposal. Enzymes and fluorophores have largely replaced radioactive isotopes as detectable tags for assays. A number of advancements in reagents and instrumentation make these newer technologies more versatile and powerful. Enzymatic tags such as horseradish peroxidase (HRP) are most commonly used for blotting, immunoassays and immunohistochemistry methods. Fluorescent tags are used predominately for cellular imaging, nucleic acid amplification and sequencing and microarrays; however, fluorescence technology is developing rapidly for application in all types of assays.

The detection of protein often involves the use of specific antibodies. Accordingly, the detection of PqsD protein or a functional variant thereof may include a specific PqsD antibody. Antibodies can be raised using well established techniques for immunizing animals with prepared forms of the antigen. A variety of reagents is available to assist in antibody production and purification, and various companies specialize in antibody production services. Depending on the application to be performed, different levels of purity and types of specificity are needed in a supplied primary antibody. To name just a few parameters, antibodies may be monoclonal or polyclonal, supplied as antiserum or affinity-purified solution, and validated for native protein or denatured protein detection.

An antibody that recognizes the target antigen, here PqsD or functional variant thereof, is called the "primary antibody." If this antibody is labeled with a tag, direct detection of the antigen is possible. Usually, however, the primary antibody is not labeled for direct detection. Instead a "secondary antibody" that has been labeled with a detectable tag is applied in a second step to probe for the primary antibody, which is bound to the target antigen. Thus, the antigen is detected indirectly. Another form of indirect detection involves using a primary or secondary antibody that is labeled with an affinity tag such as biotin. Then a secondary (or tertiary) probe, such as streptavidin that is labeled with the detectable enzyme or fluorophore tag, can be used to probe for the biotin tag to yield a detectable signal. Several variants of these probing and detection strategies exist. However, each one depends on a specific probe (e.g., a primary antibody) whose presence is linked directly or indirectly to some sort of measurable tag (e.g., an enzyme whose activity can produce a colored product upon reaction with its substrate).

Usually, a primary antibody without a detectable label and some sort of secondary (indirect) detection method is required in assay methods. Nevertheless, nearly any antibody can be labeled with biotin, HRP enzyme or one of several fluorophores if needed. Most primary antibodies are produced in mouse, rabbit or one of several other species. Nearly all of these are antibodies of the IgG class. Therefore, it is relatively easy and economical for manufacturers to produce and supply ready-to-use, labeled secondary antibodies for most applications and detection systems. Even so, several hundred options are available, differing in the level of purity, IgG- and species-specificity, and detection label. The choice of secondary antibody depends upon the species of animal in which the primary antibody was raised (the host species). For example, if the primary antibody is a mouse monoclonal antibody then the secondary antibody must be an anti-mouse antibody obtained from a host other than the mouse.

With biotin-binding proteins as probes, the highly specific affinity interaction between biotin and avidin or streptavidin protein is the basis for many kinds of detection and affinity-purification methods. Enzymatic labels are most commonly used as secondary antibody (or streptavidin) tags for detection in blotting and immunoassays. Enzymes provide detectable signal via their activity; reaction with a specific substrate chemical yields a colored, light-emitting, or fluorescent product. Examples are beta-galactosidase, luciferase, alkaline phosphatase (AP) and horseradish peroxidase (HRP). Fluorescent Labels for Detection were historically used in a small number of cell biology applications such as flow cytometry (FC), fluorescence-activated cell sorting (FACS) and immunohistochemistry (IHC) using fluorescence microscopy. Examples of common fluorophores for labeling probes are fluorescein (fluorescein isothiocyanate, FITC) and rhodamine (tetramethyl rhodamine isothiocyanate, TRITC), green fluorescent protein (GFP) and the phycobiliproteins (allophycocyanin, phycocyanin, phycoerythrin and phycoerythrocyanin).

Alternatively or additionally, two markers may be used in order to detect proximity of two substances, e.g. the test compound and the PqsD protein. The markers may be, e.g. one radioactive or fluorescent marker and one scintillator (e.g. for a scintillation proximity assay) or two fluorescent markers may be used (e.g. for FRET). In one example the PqsD protein and the test substance could be labeled with a first and a second marker. In case the test substance is bound to the protein, and the labels are therefore in close proximity, energy could be transferred from the first to the second label, thus detecting the interacting of PqsD protein and test substance. This test could be designed as a competition binding test, wherein a known PqsD ligand carries one of the labels.

Examples of suitable marker combinations include radiolabels ³H, ³³P, ³⁵S or ¹⁴C, ¹²⁵I combined with scintillator such as Yttrium silicate or polyvinyl-toluene, e.g. compartmented in a microparticle or a donor fluorescent marker such as fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives combined with a acceptor fluorescent marker such as LC-Red 610, LC -Red 640, LC-Red 670, LC - Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium).

For competition binding experiments a known ligand or binding molecule of PqsD is labeled with at least one detectable marker and added to the incubation step of b). After step b) bound labeled ligand is separated from non-bound ligand. The separation may be done by a common separation step such as filtration, centrifugation, immobilization, phase separation and removal of liquids etc. The amount of signal provided by the label is indicative for the amount of ligand bound and therefore also for the amount of test compound bound to the biomolecule, as ligand and test compound compete for the binding to the biomolecule.

In an embodiment the assay for detection of the interaction of the test compound and the PqsD protein is a SPA (scintillation proximity assay), a FRET (fluorescence resonance energy transfer) assay, TR-FRET (time-resolved fluorescence resonance energy transfer) assay or a FP (fluorescence polarisation) assay.

SPA (scintillation proximity assay) is a type of technology that is used for biochemical screening which permits the rapid and sensitive measurement of a broad range of processes biologically in a homogeneous system. The type of beads that is involved in the SPA are microscopic in size and within the beads itself, there is a scintillant which emits light when it is stimulated. Stimulation occurs when radio-labeled molecules interact with the bead. This interaction will trigger the bead to emit light, which can be detected using scintillation counters. Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically <10 nm). For monitoring binding of a protein to an agent, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Representative donor fluorescent moieties that can be used with various acceptor fluorescent moieties in FRET technology include fluorescein, Lucifer Yellow, B-phycoerythrin etc. Representative acceptor fluorescent moieties, depending upon the donor fluorescent moiety used, include LC-Red 610, LC -Red 640, LC-Red 670, LC -Red 705, Cy5, Cy5.5, Lissamine rhodamine B sulfonyl chloride, tetramethyl rhodamine isothiocyanate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO). Alternatively, time-resolved fluorescence resonance energy transfer (TR-FRET) may be used for the test system of the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro- to millisecond measurements. Fluorescence polarisation (FP)-based assays are assays which use polarized light to excite fluorescent substrate in solution. These fluorescent substrates are free in solution and tumble, causing the emitted light to become depolarised. When the substrate binds to a larger molecule, i.e. the acyl group, its tumbling rates are greatly decreased, and the emitted light remains highly polarized. Alternatively, mass spectrometry may be used.

In a further aspect of the present invention, the method is carried out in vitro. A procedure performed in vitro (Latin: within the glass) is performed not in a living organism but in a controlled environment, such as in a test tube or Petri dish. The finding of the present inventors that incubation of the substrates anthraniloyl-CoA and 3-oxodecanoic acid results in the formation of HHQ by the use of the protein PqsD allows performing of this reaction in an in-vitro assay system. This does not only open the possibility of further investigating the biosynthesis of HHQ and other HAQs, but also offers a new approach for the development of a system for screening for inhibitors of the PqsD protein. An in-vitro assay avoids disadvantages which may arise in in-vivo methods such as a possible ambiguity, for example, if components are present which are involved in the synthesis of HAQs, in particular HHQ or PQS, the work with possible pathogenic agents, the restricted possibility of automatisation and the increased consume of time and labour. In the in-vitro assay, the components participating in the reaction are combined in a test tube, the reaction is allowed to proceed and binding of components or conversion of substrate to product is determined. Consequently, the in-vitro assay is a preferred embodiment of the present invention.

Thereby, the assay of the present invention is preferably a cell-free assay comprising contacting the PqsD protein or variant thereof with a test compound and determining the ability of the test compound to bind to the PqsD protein or variant thereof. Binding of the test compound to the PqsD protein or variant thereof may be determined directly or indirectly as outlined above.

Alternatively, the skilled person may use a cell-based method for performing the enzymatic reaction using the PqsD protein. The skilled person is thereby in the condition, based on the prior art and the teaching provided therewith, to develop such methods. Thus, in an embodiment of the present invention, the test system is in a cell such as an animal cell, a bacterial cell or a fungal cell. Preferably, the cell does not comprise a quorum sensing system, more preferably the cell does not produce HAQs and even more preferably, the cell does not produce HHQ. The cell may be a genetically engineered cell, into which at least the pqsD gene or a functionally active variant thereof as described above is introduced. If the cell is transfected with only the pqsD gene, then it may be necessary to add anthraniloyl-CoA for the method to work. Alternatively, additionally a gene for producing anthraniloyl-CoA may be added, if necessary. Such genes are known to those in the art. For example, such genes may be the above mentioned 2-aminobenzoate-CoA ligase, e.g. from Azoarcus evansii (SEQ ID NO: 3), or the above mentioned PqsA gene, e.g. from *Pseudomonas aeruginosa* such as a protein with SEQ ID NO: 2. In this case, anthranilate instead of anthraniloyl-CoA is added to the test reaction. The preparation of cells and cell lines transfected with the pqsD gene and possibly further genes to allow formation of product such as the PqsA gene or the 2-aminobenzoate-CoA ligase gene is known in the art and is described herein with respect to the preparation of PqsD protein by genetic engineering.

Alternatively, cell lysates (crude, fractionated or purified) may be used. Exemplary methods for producing these are known to the skilled person and may include fragmentation, centrifugation and resuspension.

In another embodiment, the method comprises the provision of pqsD nucleic acid coding for a PqsD protein as defined above and the identification of inhibitors of pqsD expression. Inhibitors of pqsD expression are identified in a method, wherein a cell is contacted with a test compound and the expression of pqsD mRNA or protein in the cell is determined. The level of expression of pqsD mRNA or protein in the presence of the test compound is compared with the level of expression of pqsD mRNA or protein in the absence of the test compound. The test compound can then be identified as an inhibitor of pqsD expression based on this comparison, if expression of pqsD mRNA or protein is lower than in the absence of the test compound. Methods for determining of pqsD mRNA or protein are known in the art or are described herein.

The effect of the test compound on the nucleic acid may be determined on a variety of expression or signal transduction levels. The test compound could be designed to bind to a regulatory sequence of the pqsD gene or the pqsD gene itself. Thereby, the test compound could have an influence on the expression of the gene. Accordingly, the binding of test compound to the regulatory sequence could be determined by detecting the complex of the regulatory sequence or gene and the test compound. Suitable methods of detecting complexes of two or more components are detailed herein. The regulatory sequence is a segment of DNA where regulatory proteins such as transcription factors preferentially bind. These regulatory proteins bind to short stretches of DNA called regulatory regions, which are appropriately positioned in the genome, usually a short distance 'upstream' of the gene being regulated. By doing so, these regulatory proteins can recruit another protein complex, called the RNA polymerase. In this way, they control gene expression. The regulatory sequence includes the promoter region which usually works in concert with other regulatory regions to direct the level of transcription of a given gene.

Alternatively, the effect, e.g. binding, of the test compound and the influence on the gene transcription can be detected indirectly. For this, the effect downstream the pqsD gene could be detected. For example, the effect on the transcription and translation related to pqsD could be determined. In one embodiment, the amount of pqsD mRNA or PqsD protein is detected.

Suitable methods of detecting mRNA include e.g. Northern blot analysis, nuclease protection assays (NPA), in situ hybridization, and reverse transcription-polymerase chain reaction (RT-PCR) which are methods known in the art. For the Northern blotting procedure, nonisotopic or high specific activity radiolabeled probes can be used including random-primed, nick-translated, or PCR-generated DNA probes, in vitro transcribed RNA probes, and oligonucleotides for hybridization to the membrane bound pqsD nucleic acid. Additionally, sequences with only partial homology (e.g., cDNA from a different species) may be used as probes. The Nuclease Protection Assay (NPA) is an extremely sensitive method for the detection and quantitation of specific mRNAs. The basis of the NPA is solution hybridization of an antisense probe (radiolabeled or nonisotopic) to an RNA sample. In RT-PCR, an RNA template is copied into a complementary DNA (cDNA) using a retroviral reverse transcriptase. The cDNA is then amplified exponentially by PCR.

The above methods may include nucleic acids labeling. A series of techniques are known to the skilled person allowing for labeling of DNA, RNA or oligonuleotides. These include for example Nick translational labeling, random primed DNA labeling, PCR labeling of DNA probes and oligonucleotide 3'/5' end labeling, transcriptional labeling of RNA probes, oligonucleotide 3'/5' end labeling and oligonucleotide tailing. Different labels are known which may be used in the above labeling methods. Some of them including their detection are exemplarily described in the following:
Biotin-labeled compounds can be detected for example by anti-biotin antibodies or by avidin or streptavidin conjugates. Anti-biotin antibodies (e.g. monoclonal anti-biotin antibody or Fab-fragment, conjugated with alkaline phosphatase (AP)) may be used in the detection of biotin-labeled nucleic acids by enzyme immunoassay with luminescence on nylon membranes. Avidin or streptavidin conjugates are used for the detection of biotin-labeled substances (*e.g*., biotinylated antibodies) which can be used for several immunological detection systems. For this, avidin or streptavidin e.g. from Streptomyces avidinii could be coupled to alkaline phosphatase or to ß-peroxidase. Probe-target hybrids may be detected with an enzyme-linked immunoassay. This immunochemical detection step is usually more sensitive than radioactive detection procedures. In this assay, the membrane may be blocked to prevent non-specific interaction of the antibody with the filter. Alkaline phosphatase-conjugated antibody, specific for digoxigenin, recognizes the digoxigenin molecule on the labeled hybrid. Addition of an alkaline phosphatase substrate allows the visualization of the hybrids. For chemiluminescence detection, suitable substrates for alkaline phosphatase are dioxetane phenyl phosphates such as disodium 3-(4-methoxyspiro {1,2-dioxetane-3,2-(5-chloro)tricyclo [3.3.1.1^{3,7}]decan}-4-yl)phenyl phosphate or disodium 4-chloro-3-(methoxyspiro {1,2-dioxetane-3,2-(5-chloro)tricyclo [3.3.1.1^{3,7}]decan}-4-yl)phenyl phosphate. Upon dephosphorylation by alkaline phosphatase, an intermediate is formed whose decomposition results in light emission which can be recorded e.g. on X-ray film. Colorimetric detection of DIG-labeled probes is usually performed with colorless substrates which form a redox system. Examples are like 5-bromo-4-chloro-3-indolyl-phosphate and 4-Nitro-blue-tetrazolium-chloride. 5-bromo-4-chloro-3-indolyl-phosphate is oxidized by the alkaline phosphatase to indigo by release of a phosphate group. In parallel, 4-Nitro-blue-tetrazolium-chloride is reduced to diformazan. The reaction products form a water insoluble dark blue to brownish precipitate, depending on the type of membrane.

Various reporter molecules can be coupled to detecting antibodies to visualize the specific probe-target hybridization including, but not limited to, enzyme-coupled antibodies, fluorochrome-labeled antibodies (detection by fluorescent microscope and specific filters which allow visualization of the wavelength emitted by the fluorescent dye) and antibodies coupled to colloidal gold (detection by electron microscope on cryostatic sections).

Multiple simultaneous hybridizations can be performed by using combinations of digoxigenin-, biotin- and fluorochrome-labeled probes to localize different chromosomal regions or different RNA sequences in one preparation. Such multiprobe experiments are made possible by the availability of different fluorescent dyes coupled to antibodies. These include fluorescein or FITC (fluorescein isothiocyanate; yellow), rhodamine or TRITC (tetramethylrhodamine isothiocyanate; red) and AMCA (amino-methylcoumarin acetic acid; blue).

The effect on the regulatory sequence can also by detected by attaching the regulator sequence to a reporter gene and introducing the resulting DNA construct into a cell or organism. For bacteria or eukaryotic cells in culture, this is usually in the form of a circular DNA molecule called a plasmid. It is important to use a reporter gene that is not natively expressed in the cell or organism under study, since the expression of the reporter is being used as a marker for successful uptake of the gene of interest. Commonly used reporter genes that induce visually identifiable characteristics usually involve fluorescent and luminescent proteins; examples include the gene that encodes jellyfish green fluorescent protein (GFP), the enzyme luciferase and the red fluorescent protein from the gene dsRed. Another common reporter in bacteria is the lacZ gene. An example of a selectable-marker reporter in bacteria is the chloramphenicol acetyltransferase (CAT) gene. The influence of a test compound may be detected by the determining the amount of the above signal relative to a control.

In a preferred embodiment of the present invention the method is a high-through-put screening method. High-throughput screening (HTS) is a method for scientific experimentation especially used in drug discovery and relevant to the fields of biology and chemistry. Using for example robotics, data processing and control software, liquid handling devices, and sensitive detectors, High-Throughput Screening or HTS allows a researcher to quickly conduct thousands or even millions of biochemical, genetic or pharmacological tests. Through this process one can rapidly identify active compounds, antibodies or genes which modulate a particular biomolecular pathway. Usually, HTS uses automation to run a screen of an assay against a library of candidate compounds. Typical HTS screening libraries or "decks" can contain from 100,000 to more than 2,000,000 compounds. Most often, the key testing vessel of HTS is the multi-well plate or microplate. Modern microplates for HTS generally have either 96, 384, 1536, or 3456 wells. These are all multiples of 96, reflecting the original 96 well microplate with 8 x 12 9mm spaced wells. Most of the wells contain experimentally useful matter, often an aqueous solution of dimethyl sulfoxide (DMSO) and some other chemical compound, the latter of which is different for each well across the plate. The other wells may be empty, intended for use as optional experimental controls. To prepare for an assay, the researcher fills each well of the plate with some biological entity that he or she wishes to conduct the experiment upon. In the present case the test system comprising a pqsD nucleic acid or protein is to be filled in. After some incubation time has passed to allow the biological matter to absorb, bind to, or otherwise react (or fail to react) with the compounds in the wells, measurements are taken across all the plate's wells, either manually or by a machine. A specialized automated analysis machine can run a number of experiments on the wells (such as shining polarized light on them and measuring reflectivity, which can be an indication of protein binding). In this case, the machine may output the result of each experiment as a grid of numeric values, with each number mapping to the value obtained from a single well. A high-capacity analysis machine can measure dozens of plates in the space of a few minutes like this, generating thousands of experimental data points very quickly. Advantageously, the method of the present invention is carried out in a robotics system, e.g. including robotic plating and robotic liquid transfer, e.g. using microfluids, i.e. channeled structured.

In a further aspect the present invention provides the use of a pqsD nucleic acid or protein for identifying a compound for treating, reducing or preventing a pathogenic infection caused by a microorganism. With respect to the terms "pqsD nucleic acid" or "PqsD protein" and "identifying a compound for treating reducing or preventing a pathogenic infection caused by a microorganism" it is referred to the definitions provided in the context of the methods of the present invention. It is noted that the methods described above may be used for the identification.

In a further aspect of the present invention the infection which is to be treated, reduced or prevented by a compound identified by a method as outlined herein is caused by a biofilm forming microorganism which forms e.g. biofilms on or within a catheter, a drug delivery device such as a medicament or drug pump or a prosthesis and is preferably *Pseudomonas,* in particular *Pseudomonas aeruginosa.*

In the method of the present invention the test compound is useful for treating, reducing or preventing a pathogenic infection if a reduction of PqsD activity is detected. Consequently, the test compound acts as an inhibitor. The compound which acts as an inhibitor of PqsD activity may be evolved to a medicament useful for treating, reducing or preventing a pathogenic infection identified according to the present invention. For the production of the medicament the identified target or its pharmaceutically acceptable salt has to be in a pharmaceutical dosage form in general consisting of a mixture of ingredients such as pharmaceutically acceptable carriers or auxiliary substances combined to provide desirable characteristics. An example of such an identified inhibitor is 2-[(2-phenoxybiphenyl-4-carbonyl)amino]benzoic acid.

The formulation comprises at least one suitable pharmaceutically acceptable carrier or auxiliary substance. Examples of such substances are demineralised water, isotonic saline, Ringer's solution, buffers, organic or inorganic acids and bases as well as their salts, sodium chloride, sodium hydrogencarbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvans such as gelatin, dextran, cellulose and its derivatives, albumins, organic solvents, complexing agents such as citrates and urea, stabilizers, such as protease or nuclease inhibitors, preferably aprotinin, ε-aminocaproic acid or pepstatin A, preservatives such as benzyl alcohol, oxidation inhibitors such as sodium sulphite, waxes and stabilizers such as EDTA. Colouring agents, releasing agents, coating agents, sweetening, flavouring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the medicament is in general adjusted using a suitable organic or inorganic buffer, such as, for example, preferably using a phosphate buffer, Tris buffer (tris(hydroxymethyl)aminomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicaments as well as suitable pharmaceutically acceptable carrier or auxiliary substance are well known to the one of skill in the art. Pharmaceutically acceptable carriers and auxiliary substances are a. o. chosen according to the prevailing dosage form and identified compound.

The pharmaceutical composition can be manufactured for oral, nasal, rectal, parenteral, vaginal, topic or vaginal administration. Parental administration includes subcutaneous, intracutaneous, intramuscular, intravenous or intraperitoneal administration.

The medicament can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular patient will depend upon a variety of factors including the activity of the identified compound, the dosage form, the age, body weight and sex of the patient, the duration of the treatment and like factors well known in the medical arts.

The total daily dose of the compounds of this invention administered to a human or other mammal in single or in divided doses can be in amounts, for example, from about 0.01 to about 50 mg/kg body weight or more preferably from about 0.1 to about 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of the compounds of the present invention per day in single or multiple doses.

The invention is further explained by the following figures and examples which are intended to illustrate but not to limit the scope of the present invention.

### FIGURES

Figure 1 shows the chemical structures of 4-hydroxy-2-heptylquinoline (HHQ) (panel A) and *Pseudomonas* quinoline signal (PQS) (panel B).
Figure 2 shows the biosynthetic scheme for HHQ biosynthesis as proposed in the prior art. The substrates anthraniloyl-CoA and 3-oxodecanoic acid are converted via an assumed intermediate to HHQ. The role of the PqsB, PqsC and PqsD proteins in the biosynthesis remains unclear.
Figure 3A shows the HPLC-MS chromatogram, MS- and MS²-spectrum of the HHQ reference substance.
Figure 3B shows the HPLC-MS chromatogram, MS- and MS²-spectrum of the PqsD assay. It is obvious that the compound produced by the PqsD assay is HHQ. BPC stands for base peak chromatogram, EIC stands for extracted ion chromatogram.
Figure 4 shows kinetic parameters of PqsD at fixed anthraniloyl-CoA and varied 3-oxodecanoic acid concentrations. It is shown that the PqsD protein fits to Michaelis-Menten. The Kₘ value was determined to be 0.56 mM.
Figure 5 shows the inhibition of HHQ formation by 2-[(2-phenoxybiphenyl-4-carbonyl)amino]benzoic acid. The IC₅₀ value was determined to be 35 µM.
Figure 6 shows the chemical structure of 2-[(2-phenoxybiphenyl-4-carbonyl)amino]benzoic acid, identified as an inhibitor of the PqsD protein.

### EXAMPLES

### Example 1: Formation of HHQ by the PqsD protein

Experimental protocol. The structure of HHQ suggests that its formation takes place by the condensation of anthranilic acid or its CoA derivative with 3-oxodecanoic acid (Figure 2). This theory was further corraborated by feeding studies with labelled acetate which clearly showed that the heterocyclic part of the quinoline ring system must also be synthesized from acetate units (Bredenbruch F. et al. J Bacteriol. 2005; 187: 3630-3635). The carbon-carbon bond formation between carbons 3 and 4 of the quinoline ring is likely to be catalyzed by one of the proteins PqsB, PqsC or PqsD which show all homology to the β-ketoacyl-acyl carrier protein synthase III family. To test of whether PqsD is necessary for HHQ formation, we purified recombinant PqsD protein and tried to readjust HHQ formation *in vitro.* The heterologous protein was incubated with anthraniloyl-CoA and 3-oxodecanoic acid and the reaction was analyzed by HPLC-MS.

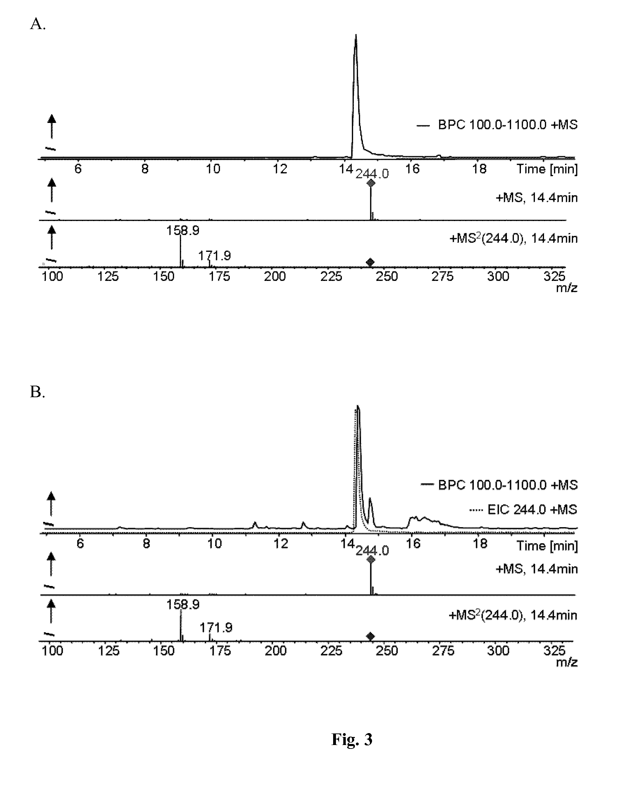

Results. The formation of HHQ in this assay was confirmed by comparison to HHQ reference substance, which eluted at the same retention time (14.4 min) and exhibited identical mass spectrum and fragmentation pattern (Figure 3A and 3B). A quantification method for HHQ by HPLC-MS² was developed, based on the most abundant fragment ion of HHQ at 159 Da. Thereby the amount of HHQ formed in the *in vitro* assay could be determined.

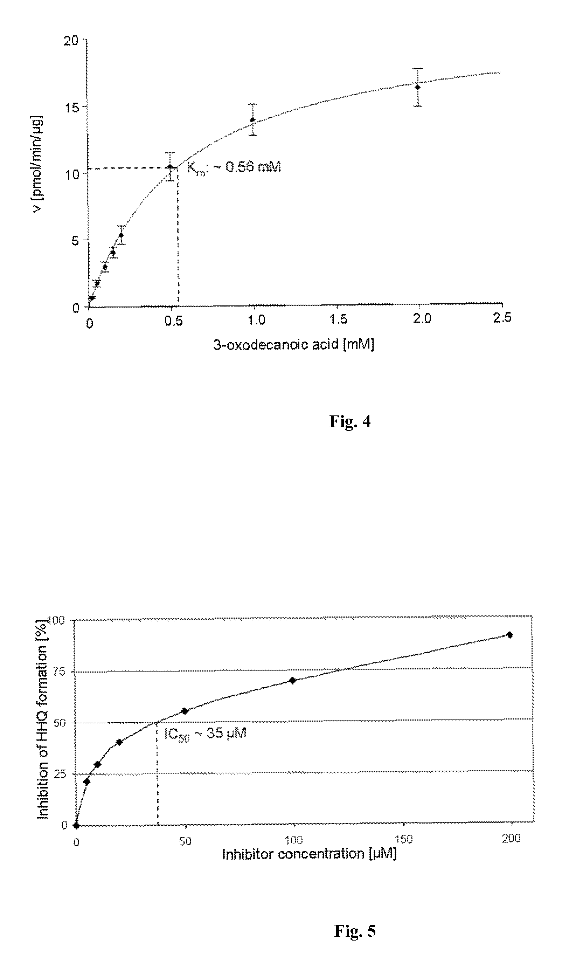

The kinetic parameters of PqsD with varying concentrations of 3-oxodecanoic acid at a fixed concentration of anthraniloyl-CoA of 200 µM were assigned. The *Kₘ* value of PqsD for 3-oxodecanoic acid under this condition was 562 ± 91 µM (Figure 4).

### Example 2: Determination of PqsD inhibitors in vitro

Experimental protocol. With the PqsD assay established so far, it is possible to screen for possible inhibitors of HHQ formation by PqsD *in vitro.* For this purpose the concentrations of anthraniloyl-CoA, 3-oxodecanoic acid and PqsD protein are assigned to fixed values. At first PqsD, anthraniloyl-CoA and the compound to be tested are preincubated for a fixed period. The putative inhibitor is added to the assay in increasing concentrations. The reaction is started by the addition of 3-oxodecanoic acid and incubated for a fixed period. Then the reaction is quenched by the addition of methanol to a final concentration of 50 percent. For each reaction the HHQ formation is quantified by HPLC/MS². By comparing the amounts of HHQ formed in presence of the different concentrations of the inhibitor to that of a control reaction without inhibitor, it can be calculated for the inhibitor concentration at which HHQ formation is 50 percent of the control reaction (IC₅₀).

2-[(2-phenoxybiphenyl-4-carbonyl)amino]benzoic acid, a known inhibitor of bacterial FabH (Nie Z. et al., J. Med. Chem. 2005; 48: 1596-1609), was tested with PqsD in concentrations of 5, 10, 20, 50, 100 and 200 µM.

Results. The IC₅₀ of 2-[(2-phenoxybiphenyl-4-carbonyl)amino]benzoic acid for HHQ formation by PqsD was determined to be in the range of ∼ 35µM (Figure 5). This finding serves as experimental proof of principle for using this assay in the screening of potential inhibitors of PqsD. It enables for inhibitor screening *in vitro,* without the potential ambiguity of *in vivo* experiments. Testing the inhibitors *in vivo* implies working with the pathogen strain. Although *in vivo* testing of good inhibitor candidates is indispensable, it is undesirable if having to screen a whole substance library *in vivo.* This is the huge advantage of the presented *in vitro* assay. It delivers fast results, which are not influenced by the other interconnected quorum sensing systems in the bacterium.

### Experimental protocol

*Materials:* Anthranilic acid was purchased from Sigma-Aldrich. Coenzyme A (CoA) trilithium salt was purchased from AppliChem. 4-Hydroxy-2-heptylquinoline was supplied by Susanne Häusler from the Department of Chronical Pseudomonas Infections at the Helmholtz-Centre for Infection Research, Braunschweig (Germany). Ethyl 3-oxodecanoate was purchased from Pharmten Chemical Co. (China).

*Synthesis of 3-oxo-decanoic acid:* A 2.05 M aqueous solution of NaOH (2.5 mmol in 1.22 ml H₂O) was added at room temperature to ethyl 3-oxo-decanoate (215 mg, 1.00 mmol) and the mixture was stirred over night. After diluting with water the alkaline solution (pH 12-13) was extracted with ethyl ether which was discarded. The aqueous layer was acidified with 1 M aqueous HCl to pH 1 and the free acid was extracted with dichloromethane³. The organic layer was dried over Na₂SO₄ and evaporated to yield the β-keto acid as white solid (163 mg, 0.875 mmol, 88 %).¹H-NMR (400 MHz, CDCl₃, keto:enol 84:16): keto: δ = 0.86 (t, ³*J*_{10,9} = 6.91 Hz, 3 H, 10-H), 1.21-1.32 (m, 8 H, 6-H-8-H), 1.52-1.64 (m, 2 H, 5-H), 2.54 (t, ³*J*_{4,5} = 7.39 Hz, 2 H, 4-H), 3.49 (s, 2 H, 2-H). enol (selected signals): δ = 2.21 (t, ³*J*_{4,5} = 7.62 Hz, 2 H, 4-H), 5.01 (s, 1 H, 2-H), 11.80 (s, 1 H, OH).

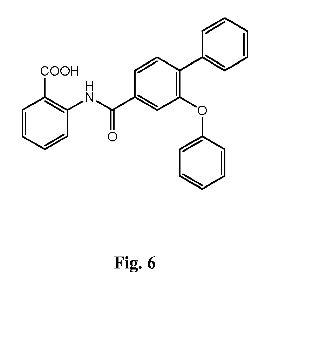

*Synthesis of 2-[(2-phenoxybiphenyl-4-carbonyl)amino]benzoic acid:* Synthesis was performed as described by Nie Z. et al. (J. Med. Chem. 2005; 48: 1596-1609). 1H NMR (300 MHz, DMSO-d6) δ 12.27 (s, 1H), 8.68 (d, *J*) 8.4 Hz, 1H), 8.08 (dd, *J*) 1.5 and 7.9 Hz, 1H), 7.88 (dd, *J*) 1.7 and 8.1 Hz, 1H), 7.77 (d, J) 8.0 Hz, 1H), 7.38-7.69 (m, 9H), 7.26 (t, *J*) 7.8 Hz, 1H), 7.17 (t, *J*) 7.9 Hz, 1H), 7.05 (m, 2H). Figure 6 shows the chemical structure of 2-[(2-phenoxybiphenyl-4-carbonyl)amino]benzoic acid.

*Synthesis of anthraniloyl-CoA:* Anthraniloyl-CoA was created in vitro by a 2-aminobenzoate-CoA ligase from *Azoarcus evansii* from anthranilic acid and CoA as described below.

*Bacterial strains, plasmids and media:* Genomic DNA from *Pseudomonas aeruginosa* UCBPP-PA14 (Rahme L.G. et al., Science 1995, 268:1899-1902) was obtained from Susanne Häusler from the Department of Chronical Pseudomonas Infections at the Helmholtz-Centre for Infection Research, Braunschweig (Germany). *Escherichia coli* DH10B (Invitrogen) and plasmid pET28b(+) (Novagen) were used for DNA manipulations. *E. coli* BL21 (DE3) from Stratagene was used for expression of N-terminal His-tagged proteins. Media and growth conditions were according to standard protocols as described by Sambrook et al. (Molecular cloning:A laboratory manual. Cold Spring Harbor, NY: Cold Spring Harbor Laboratory Press, 2001)

*Construction of plasmids:* Restriction enzymes and dNTPs were purchased by Fermentas. Phusion polymerase from Finnzymes was used for PCR reactions. Conditions for PCR reactions were: Initial denaturation step: 2 min 95°C, denaturation 18 sec 95°C, annealing 14 sec 65°C, elongation 25 sec 72°C, cycle 30 times, terminal elongation step 5 min 72°C. Primers were designed with a four base pair overhang 5' of the restriction sites. PCR products and pET28b(+) plasmid were double digested with the respective restriction enzymes for 2 h at 37°C. Digested DNA fragments were purified by gel electrophoresis on 0.9 % agarose gels and cut out after visualisation using SYBR Green dye (Invitrogen). DNA was eluted from the gels using the NucleoSpin Extract II kit from Macherey-Nagel according to the manufacturers instructions.

Construction of pET28b(+)-pqsD ― pqsD was amplified by PCR with primer l, 5'-CTAGCATATGGGTAATCCGATCCTGGC-3' (underlined letters indicate a Ndel site; SEQ ID NO: 4), and primer II, 5'-CTAGGAATTCTCAACATGGCCGGTTCACCT-3' (underlined letters indicate a EcoRl site; SEQ ID NO: 5). The 1-kb Ndel-EcoRl fragment of pqsD was cloned between the Ndel and EcoRI sites of pET28b(+), resulting in pET28b(+)-pqsD.

Construction of pET28b(+)-2-aminobenzoate-CoA ligase *A. evansii -* 2-aminobenzoate-CoA ligase *A. evansii* was amplified by PCR with primer 1, 5'-GTTACATATGGTGACGAGTCACGTCGA-3' (underlined letters indicate a Ndel site; SEQ ID NO:6), and primer II, 5'-GTTAAAGCTTTCAGGGTTTCCCCTCGCGCA-3' (underlined letters indicate a HindIII site; SEQ ID NO: 7). The 1.6-kb Ndel-HindIII fragment of 2-aminobenzoate-CoA ligase *A. evansii* was cloned between the Ndel and HindIII sites of pET28b(+), resulting in pET28b(+)-2-aminobenzoate-CoA ligase

### A. evansii

*Production and purification of recombinant PqsD and 2-aminobenzoate-CoA ligase A. evansii in E. Coli:* For production of PqsD and 2-aminobenzoate-CoA ligase A. *evansii as* N-terminal His-tagged fusion proteins, *E. coli* BL21 (DE3) harboring pET28b(+)-pqsD or pET28b(+)-2-aminobenzoate-CoA ligase *A. evansii* respectively, was grown at 37°C in 250 ml LB medium containing 50 µg ml⁻¹ kanamycin to an OD₆₀₀ of approximately 0.8 units. For protein expression cultures were induced with 0.2 mM isopropyl β-D-thiogalactopyranoside (IPTG) and incubated at 16 °C for 16 h. The cells were harvested by centrifugation at 7300 X g for 12 min at 4 °C in a Beckman Coulter Avanti J-E centrifuge. All following steps were carried out at 4 °C. The cell pellet was resuspended in 25 ml binding buffer (50 mM Tris-HCl, pH 8.0, 150 mM NaCl, 20 mM imidazole, glycerol 10 % (*v*/*v*)) and lysed with a French press. Cell debris was removed by centrifugation at 25000 X g for 45 min. The supernatant was filtered through 1.2 µm Acrodisc syringe filters (PALL life Sciences). The filtered supernatant (25 ml) was loaded onto a 1 ml Hi-Trap His column using the Akta purifier system (both from GE Healthcare) at a flowrate of 1 ml min⁻¹. The unbound sample was washed away with 5 column volumes binding buffer. For elution of the His-tagged heterologous proteins a stepwise gradient with 7%, 16 %, 40 % and 100 % elution buffer (50 mM Tris-HCl, pH 8.0, 150 mM NaCl, 500 mM imidazole, glycerol 10 % (*v*/*v*)) with 6 column volumes for each step was applied. The fractions containing heterologous protein were buffer exchanged to 50 mM Tris-HCl, pH 8.0, 150 mM NaCl, glycerol 10 % (v/v) using PD10 columns (Amersham Biosciences) and further concentrated using Amicon ultracentrifugal filter devices (30 kDa, Millipore) at 3200 X g. PqsD was obtained at a final concentration of 5 mg ml⁻¹. 2-aminobenzoate-CoA ligase *A. evansii* was obtained at a final concentration of 2 mg ml⁻¹. Aliquots of 50 µl were frozen in liquid nitrogen and stored at -80 °C.

*Protein analysis:* Protein concentrations were determined with Bio-Rad protein assay kit according to the manufacturers instructions using bovine serum albumin (BSA) as a standard (0.5 - 8 µg ml⁻¹). Protein fractions were separated on a 12 % sodium dodecyl sulfate (SDS)-polyacrylamide gel by electrophoresis. Proteins were visualized in the gel by staining with Coomassie Brilliant Blue R-250. For matrix assisted laser desorption ionisation time of flight mass spectrometry (MALDI-ToF-MS), protein bands were cut from the SDS-polyacrylamide gel and in gel digestion with trypsin was performed. Samples were prepared using Zip-Tips (Millipore).

*Formation and isolation of anthraniloyl-CoA created in vitro by 2-aminobenzoate-CoA ligase A. evansii:* 5 mg anthranilic acid was dissolved in 1.2 ml 100 mM Tris-HCl, pH 8.0. To this solution 150 µl MgCl₂ (0.1 M), 250 µl ATP (0.1 M), 250 µl coenzyme A (CoASH) (0.1 M), and 150 µl 2-aminobenzoate-CoA ligase *A. evansii* (2 mg ml⁻¹) were added. The reaction mixture was incubated at 37° for 3 h. Water was removed by freeze drying and the remainder was dissolved in 500 µl of a mixture of 50 % water and 50 % methanol (*v*/*v*). Purification was performed using a preparative HPLC system from Waters consisting of the 2545 binary gradient module, the 515 HPLC pump for the make-up flow, the SFO system fluidics organizer, the 2998 PDA, the 3100 mass detector and the 2767 sample manager, operating with a X-Bridge RP-C₁₈ column (19x100 mm, 5 µM). Solvents were aqueous 20 mM ammonium formate buffer and methanol with 5 % aqueous 20 mM ammonium formiate buffer. Gradient starts at 90 % aqueous buffer with hold for 3 min and increases then to 95 % organic solvent within 15 min. Flow rate was 25 ml min⁻¹. Fractions were collected utilising mass-guided fraction collection with trigger mass of 887.6 Da.

HHQ quantification by HPLC-MS²- HPLC-MS analysis was carried out using a HPLC system from the Agilent 1100 series coupled to a Brucker Daltonics HCTultra ESI-MS iontrap instrument operated in positive ionization mode. For separation a Onyx Monolythic C18 column (50x2 mm, Phenomenex) was used. Flow was 400 µl min⁻¹ starting at 10 % acetonitrile, hold for 1.5 min, then increased to 65 % acetonitrile over 5 min and finally ramped to 95 % acetonitrile in 0.5 min. For HHQ quantification the signal intensities of fragment ion 159.0 Da created by fragmentation of 244.0 Da were used. For creation of the standard curve HHQ standards containing 1, 5, 10, 20, 50,100 and 200 ng ml⁻¹ were measured.

*Determination of kinetic parameters of PqsD:* The reaction mixtures contained 50 mM Tris-HCl, pH 8.0, PqsD protein (1 µg), 0.2 mM anthraniloyl-CoA and 3-oxodecanoic acid ranging in concentration from 0.02 to 2 mM in a total volume of 50 µl. All components except 3-oxodecanoic acid were mixed and temperature equilibrated for 2 min at 37 °C. Reactions were started by addition of 3-oxodecanoic acid and incubated at 37 °C for 2 min. Previous variations of incubation time guaranteed steady -state parameters over this period. Reactions were stopped by addition of 50 µl methanol. HHQ was quantified by HPLC by injecting 10 µl of the reaction mixture. Reactions for all tested concentrations of 3-oxodecanoic acid were performed in triplicates.

*Determination of inhibition of HHQ formation:* The reaction mixtures contained 50 mM Tris-HCl, pH 8.0, PqsD protein (1 µg), 0.2 mM anthraniloyl-CoA, 0.35 mM 3-oxodecanoic acid and 2-[(2-phenoxybiphenyl-4-carbonyl)amino]benzoic acid ranging in concentration from 5 over 10, 20, 50, 100 to 200 µM in a total volume of 50 µl. The inhibitor was preincubated with all components except 3-oxodecanoic acid for 2 min at 37 °C. Reactions were started by addition of 3-oxodecanoic acid and incubated at 37 °C for 2 min. Reactions were stopped by addition of 50 µl methanol. HHQ was quantified by HPLC by injecting 10 µl of the reaction mixture. Control reactions without the inhibitor were performed in parallel and the amount of HHQ formed was set to 100 %.

### REFERENCES

Bera A.K. et al., Biochemistry 2009; 48: 8644-8655
Bredenbruch F. et al., J Bacteriol. 2005; 187: 3630-3635
Cao H. et al., Proc. Natl. Acad. Sci. USA 2001; 98: 14613-14618
Deziel E. et al., Proc. Natl. Acad. Sci. USA 2004, 101: 1339-1344
Fletcher et al., Nature Protocols 2:1254-1262 (2007)
Lesic B. et al., PLoS Pathog. 2007; 3: 1229-1239
Ni N. et al., Med. Res. Rev. 2009; 29: 65-124
Nie Z. et al., J. Med. Chem. 2005; 48: 1596-1609
Rahme L.G. et al., Science 1995, 268:1899-1902
Sambrook et al., Molecular cloning: A laboratory manual. Cold Spring Harbor, NY: Cold Spring Harbor Laboratory Press, 2001

## Claims

1. A method of identifying a compound for treating, reducing or preventing a pathogenic infection caused by a microorganism, the method comprising the steps of:
a) providing a pqsD nucleic acid or a PqsD protein or a functional fragment or variant thereof,
b) contacting the pqsD nucleic acid or the PqsD protein or a functional fragment or variant thereof with a test compound,
c) determining the binding of the test compound to the pqsD nucleic acid or the PqsD protein or a functional fragment or variant thereof,
wherein the test compound is identified as a potential compound useful for treating, reducing or preventing a pathogenic infection, when the test compound binds to the pqsD nucleic acid or the PqsD protein or a functional fragment or variant thereof.

2. The method of claim 1, wherein determining the binding of the test compound to the pqsD nucleic acid or the PqsD protein or a functional fragment or variant thereof comprises determining the activity of the PqsD protein or a functional fragment or variant thereof, wherein the test compound is identified as a potential compound useful for treating, reducing or preventing a pathogenic infection, when a reduction of the activity of the PqsD protein or a functional fragment or variant thereof in the test system relative to a control is detected.

3. The method of claim 1 or 2, wherein the pqsD nucleic acid or the PqsD protein or a functional fragment or variant thereof is from a *Pseudomonas* species, particularly *Pseudomonas aeruginosa.*

4. The method of claim 2 or 3, wherein the activity of the PqsD protein or a functional fragment or variant thereof is determined by measuring the conversion of a substrate of the PqsD protein into a product.

5. The method of claim 4, wherein the substrate is anthraniloyl-CoA and/or 3-oxo-(C₄-C₂₃)alkanoic acid and the product is 4-hydroxy-2-(C₁-C₂₀)alkyl quinoline.

6. The method of claim 5, wherein 3-oxo-(C₄-C₂₃)alkanoic acid is 3-oxodecanoic acid and the product is HHQ (4-hydroxy-2-heptyl quinoline).

7. The method of claim 5 or 6, wherein anthraniloyl-CoA is synthesized from anthralinic acid by use of 2-aminobenzoate-CoA ligase or a functional fragment or variant thereof.

8. The method of any one of claims 1 to 7, wherein the method is an *in vitro* method.

9. The method of any one of claims 1 to 8, wherein the method is carried out in an automated format, particularly a high-through-put format.

10. Use of a pqsD nucleic acid or a PqsD protein or a functional fragment or variant thereof for identifying a compound useful for treating, reducing or preventing a pathogenic infection caused by a microorganism.

11. The method of any one of claims 1 to 9 or the use of claim 10, wherein the microorganism forms a biofilm.

12. The method or use of claim 11 wherein the biofilm is formed on or within a catheter, a medicament, drug pump, or prosthesis.

13. The method of any one of claims 1 to 9, 11 or 12 or the use of any one of claims 10 to 12, wherein the microorganism is *Pseudomonas,* in particular *Pseudomonas aeruginosa.*

14. A method of producing a medicament, the method comprising the steps of:
a) identifying a test compound carrying out the method of any one of claims 1 to 9 or 11 to 13,
b) providing adequate amounts of the test compound,
c) formulating the test compound with a pharmaceutically acceptable carrier or auxiliary substance.

15. 2-[(2-phenoxybiphenyl-4-carbonyl)amino]benzoic acid for use in the treatment of a pathogenic infection caused by a microorganism as defined in any one of claims 1 or 11 to 13.


SEQUENCE LISTING

<110>  sanofi-aventis

<120>  Methods and uses relating to the identification of compound
       associated with bacterial infection

<130>  DE2010/154

<160>  7

<170>  PatentIn version 3.5

<210>  1
<211>  337
<212>  PRT
<213>  Pseudomonas aeruginosa UCBPP-PA14

<400>  1

Met Gly Asn Pro Ile Leu Ala Gly Leu Gly Phe Ser Leu Pro Lys Arg
1               5                   10                  15

Gln Val Ser Asn His Asp Leu Val Gly Arg Ile Asn Thr Ser Asp Glu
            20                  25                  30

Phe Ile Val Glu Arg Thr Gly Val Arg Thr Arg Tyr His Val Glu Pro
        35                  40                  45

Glu Gln Ala Val Ser Ala Leu Met Val Pro Ala Ala Arg Gln Ala Ile
    50                  55                  60

Glu Ala Ala Gly Leu Leu Pro Glu Asp Ile Asp Leu Leu Leu Val Asn
65                  70                  75                  80

Thr Leu Ser Pro Asp His His Asp Pro Ser Gln Ala Cys Leu Ile Gln
                85                  90                  95

Pro Leu Leu Gly Leu Arg His Ile Pro Val Leu Asp Ile Arg Ala Gln
            100                 105                 110

Cys Ser Gly Leu Leu Tyr Gly Leu Gln Met Ala Arg Gly Gln Ile Leu
        115                 120                 125

Ala Gly Leu Ala Arg His Val Leu Val Val Cys Gly Glu Val Leu Ser
    130                 135                 140

Lys Arg Met Asp Cys Ser Asp Arg Gly Arg Asn Leu Ser Ile Leu Leu
145                 150                 155                 160

Gly Asp Gly Ala Gly Ala Val Val Val Ser Ala Gly Glu Ser Leu Glu
                165                 170                 175

Asp Gly Leu Leu Asp Leu Arg Leu Gly Ala Asp Gly Asn Tyr Phe Asp
            180                 185                 190

Leu Leu Met Thr Ala Ala Pro Gly Ser Ala Ser Pro Thr Phe Leu Asp

195                         200                         205

Glu Asn Val Leu Arg Glu Gly Gly Gly Glu Phe Leu Met Arg Gly Arg
    210                         215                         220

Pro Met Phe Glu His Ala Ser Gln Thr Leu Val Arg Ile Ala Gly Glu
225                         230                         235                         240

Met Leu Val Ala His Glu Leu Thr Leu Asp Asp Ile Asp His Val Ile
                        245                         250                         255

Cys His Gln Pro Asn Leu Arg Ile Leu Asp Ala Val Gln Glu Gln Leu
                260                         265                         270

Gly Ile Pro Gln His Lys Phe Ala Val Thr Val Asp Arg Leu Gly Asn
            275                         280                         285

Met Ala Ser Ala Ser Thr Pro Val Thr Leu Ala Met Phe Trp Pro Asp
    290                         295                         300

Ile Gln Pro Gly Gln Arg Val Leu Val Leu Thr Tyr Gly Ser Gly Ala
305                         310                         315                         320

Thr Trp Gly Ala Ala Leu Tyr Arg Lys Pro Glu Glu Val Asn Arg Pro
                        325                         330                         335

Cys

<210>  2
<211>  517
<212>  PRT
<213>  Pseudomonas aeruginosa UCBPP-PA14

<400>  2

Met Ser Thr Leu Ala Asn Leu Thr Glu Val Leu Phe Arg Leu Asp Phe
1                   5                   10                  15

Asp Pro Asp Thr Ala Val Tyr His Tyr Arg Gly Gln Thr Leu Ser Arg
            20                  25                  30

Leu Gln Cys Arg Thr Tyr Ile Leu Ser Gln Ala Ser Gln Leu Ala Arg
        35                  40                  45

Leu Leu Lys Pro Gly Asp Arg Val Val Leu Ala Leu Asn Asp Ser Pro
    50                  55                  60

Ser Leu Ala Cys Leu Phe Leu Ala Cys Ile Ala Val Gly Ala Ile Pro
65                  70                  75                  80

Thr Val Ile Asn Pro Lys Ser Arg Glu Gln Ala Leu Ala Asp Ile Ala
                85                  90                  95

Ala Asp Cys Gln Ala Ser Leu Val Val Arg Glu Ala Asp Ala Pro Ser
            100                 105                 110

Leu Ser Gly Pro Leu Ala Pro Leu Thr Leu Arg Ala Ala Ala Gly Arg
        115                 120                 125

Pro Leu Leu Asp Asp Phe Ser Leu Asp Ala Leu Val Gly Pro Ala Asp
    130                 135                 140

Leu Asp Trp Ser Ala Phe His Arg Gln Asp Pro Ala Ala Ala Cys Phe
145                 150                 155                 160

Leu Gln Tyr Thr Ser Gly Ser Thr Gly Ala Pro Lys Gly Val Met His
                165                 170                 175

Ser Leu Arg Asn Thr Leu Gly Phe Cys Arg Ala Phe Ala Thr Glu Leu
            180                 185                 190

Leu Ala Leu Gln Ala Gly Asp Arg Leu Tyr Ser Ile Pro Lys Met Phe
        195                 200                 205

Phe Gly Tyr Gly Met Gly Asn Ser Leu Phe Phe Pro Trp Phe Ser Gly
    210                 215                 220

Ala Ser Ala Leu Leu Asp Asp Thr Trp Pro Ser Pro Glu Arg Val Leu
225                 230                 235                 240

Glu Asn Leu Val Ala Phe Arg Pro Arg Val Leu Phe Gly Val Pro Ala
                245                 250                 255

Ile Tyr Ala Ser Leu Arg Ala Gln Ala Arg Ala Leu Leu Ser Ser Val
            260                 265                 270

Arg Leu Ala Phe Ser Ala Gly Ser Pro Leu Pro Arg Gly Glu Phe Glu
        275                 280                 285

Phe Trp Ala Ala His Gly Leu Glu Ile Cys Asp Gly Ile Gly Ala Thr
    290                 295                 300

Glu Val Gly His Val Phe Leu Ala Asn Arg Pro Gly Gln Ala Arg Ala
305                 310                 315                 320

Asp Ser Thr Gly Leu Pro Leu Pro Gly Tyr Glu Cys Arg Leu Val Asp
                325                 330                 335

Arg Glu Gly His Thr Ile Glu Glu Ala Gly Gln Gln Gly Val Leu Leu
            340                 345                 350

Val Arg Gly Pro Gly Leu Ser Pro Gly Tyr Trp Arg Ala Ser Glu Glu
        355                 360                 365

Gln Gln Ala Arg Phe Ala Gly Gly Trp Tyr Arg Thr Gly Asp Leu Phe
    370                 375                 380

Glu Arg Asp Glu Ser Gly Ala Tyr Arg His Cys Gly Arg Glu Asp Asp
385                 390                 395                 400

Leu Phe Lys Val Asn Gly Arg Trp Val Val Pro Thr Gln Val Glu Gln
                405                 410                 415

Ala Ile Cys Arg His Leu Pro Glu Val Ser Glu Ala Val Leu Val Pro
            420                 425                 430

Thr Cys Arg Leu His Asp Gly Leu Arg Pro Thr Leu Phe Val Thr Leu
        435                 440                 445

Ala Thr Pro Leu Asp Asp Asn Gln Ile Leu Leu Ala Gln Arg Ile Asp
    450                 455                 460

Gln His Leu Ala Glu Gln Ile Pro Ser His Met Leu Pro Ser Gln Leu
465                 470                 475                 480

His Val Leu Pro Ala Leu Pro Arg Asn Asp Asn Gly Lys Leu Ala Arg
                485                 490                 495

Ala Glu Leu Arg His Leu Ala Asp Thr Leu Tyr His Asp Asn Leu Pro
            500                 505                 510

Glu Glu Arg Ala Cys
        515

<210>  3
<211>  542
<212>  PRT
<213>  Azoarcus evansii

<400>  3

Met Thr Ser His Val Asp Thr Phe Ala Arg Asp Arg Leu Pro Pro Pro
1               5                   10                  15

Glu Gln Gln Pro Glu Phe Leu Phe Glu Leu Pro Ser Leu Gln Phe Pro
            20                  25                  30

Pro Leu Met Asn Cys Ala Val Glu Leu Leu Asp Arg Arg Val Leu Gly
        35                  40                  45

Gly Glu Gly Glu Arg Val Cys Leu Arg Ala Pro Gly Gly Leu Arg Trp
    50                  55                  60

Thr Tyr Arg Asp Leu Leu Gly His Ala Asn Arg Ile Ala Asn Val Leu
65                  70                  75                  80

Val His Glu Leu Gly Val Val Pro Gly Asn Arg Val Leu Leu Arg Gly
                85                  90                  95

Pro Asn Ser Pro Met Leu Ala Ala Cys Trp Phe Ala Ile Met Lys Ala
            100                 105                 110

Gly Ala Ile Ala Val Ala Thr Met Pro Leu Leu Arg Ala Lys Glu Leu
        115                 120                 125

Gly Gln Ile Leu Asp Lys Gly Arg Ile Thr His Ala Leu Cys Ala His
    130                 135                 140

Ala Leu Arg Gly Glu Leu Asp Glu Ala Val Ala Thr Arg Pro Ser Val
145                 150                 155                 160

Ala His Val Val Ser Phe Gly Asp Pro Ala Gly Ala Gly Leu Glu Ala
                165                 170                 175

Ala Met Ala Arg Gln Ser Gly Glu Phe Asp Asn Val Ala Thr Ala Ser
            180                 185                 190

Asp Asp Thr Cys Ile Leu Ala Phe Thr Ser Gly Thr Thr Gly Gln Pro
        195                 200                 205

Lys Ala Thr Met His Phe His Arg Asp Val Ile Ala Ala Cys Arg Cys
    210                 215                 220

Trp Pro Pro His Val Leu Arg Pro Gln Pro Asp Asp Val Phe Ile Gly
225                 230                 235                 240

Ser Pro Pro Leu Ala Phe Thr Phe Gly Leu Gly Gly Met Leu Leu Phe
                245                 250                 255

Pro Met Ser Val Gly Ala Ser Thr Val Leu Leu Glu Gln Ala Ser Pro
            260                 265                 270

Pro Lys Leu Leu Asp Ala Ile Gly Glu Phe Gly Ala Thr Ile Leu Phe
        275                 280                 285

Thr Ala Pro Thr Ser Tyr Arg Ala Met Ala Glu Gly Ala Arg Glu Arg
    290                 295                 300

Arg Leu Gly Ala Pro Leu Gly Gly Pro Leu Val Lys Cys Val Ser Ala
305                 310                 315                 320

Gly Glu Val Leu Pro Ala Ala Thr Arg Ala Leu Trp Lys Asp Ala Thr
                325                 330                 335

Gly Ile Glu Ile Ile Asp Gly Ile Gly Ala Thr Glu Met Phe His Ile
            340                 345                 350

Phe Ile Ser Ala Asp Glu Glu His Ala Arg Pro Gly Ala Thr Gly Thr
        355                 360                 365

Val Val Pro Gly Tyr Arg Ala Arg Ile Val Asp Asp Glu Gly Arg Glu
    370                 375                 380

Val Pro Ala Gly Thr Val Gly Arg Leu Ala Val Lys Gly Pro Thr Gly
385                 390                 395                 400

Cys Arg Tyr Leu Asp Asp Ser Arg Gln Arg Asn Tyr Val Gly Asp Gly
                405                 410                 415

Trp Asn Tyr Thr Gly Asp Ala Tyr Tyr Met Asp Ala Asp Gly Tyr Phe
            420                 425                 430

His Tyr Gln Ser Arg Leu Asp Asp Met Ile Val Ser Ala Gly Tyr Asn
        435                 440                 445

Ile Gly Ala Pro Glu Val Glu Asp Ala Leu Met Gln His Pro Ala Val
    450                 455                 460

Ala Glu Cys Ala Val Ile Gly Val Pro Asp Glu Glu Arg Gly Gln Ile
465                 470                 475                 480

Val Lys Ala Phe Val Val Pro Arg Pro Gly His Gly Ala Gly Glu Leu
                485                 490                 495

Leu Val Arg Glu Leu Gln Asp Phe Val Lys Arg Thr Ile Ala Pro Tyr
            500                 505                 510

Lys Tyr Pro Arg Ala Ile Glu Phe Arg Asp Ser Leu Pro Arg Thr Glu
        515                 520                 525

Thr Gly Lys Leu Gln Arg Phe Arg Leu Arg Glu Gly Lys Pro
    530                 535                 540

<210>  4
<211>  27
<212>  DNA
<213>  artificial sequence

<220>
<223>  Primer for amplification of pqsD gene

<400>  4
ctagcatatg ggtaatccga tcctggc                                          27

<210>  5
<211>  30
<212>  DNA
<213>  artificial sequence

<220>
<223>  Primer for amplification of pqsD gene

<400>  5
ctaggaattc tcaacatggc cggttcacct                                       30

<210>  6
<211>  27
<212>  DNA
<213>  artificial sequence

<220>
<223>  Primer for amplification of 2-aminobenzoate-CoA ligase gene

<400>  6
gttacatatg gtgacgagtc acgtcga                                          27


<210>  7
<211>  30
<212>  DNA
<213>  artificial sequence

<220>
<223>  Primer for amplification of 2-aminobenzoate-CoA ligase gene

<400>  7
   gttaaagctt tcagggtttc ccctcgcgca                                        30